(19) Europäisches Patentamt
European Patent Office
Office européen des brevets

(11) **EP 4 328 233 A1**

(12) **EUROPEAN PATENT APPLICATION**
published in accordance with Art. 153(4) EPC

(43) Date of publication:
**28.02.2024 Bulletin 2024/09**

(21) Application number: **22841955.2**

(22) Date of filing: **29.06.2022**

(51) International Patent Classification (IPC):
*C07K 1/02* (2006.01)  *C07K 1/14* (2006.01)
*C07K 5/09* (2006.01)  *C12N 5/071* (2010.01)
*C12N 1/00* (2006.01)  *C07K 14/78* (2006.01)

(52) Cooperative Patent Classification (CPC):
**C07K 1/02; C07K 1/14; C07K 5/0815; C07K 14/78;
C12N 1/00; C12N 5/06**

(86) International application number:
**PCT/JP2022/026062**

(87) International publication number:
**WO 2023/286611 (19.01.2023 Gazette 2023/03)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.07.2021 JP 2021115115
18.11.2021 JP 2021187740**

(71) Applicants:
• **TOPPAN INC.**
**Tokyo 110-0016 (JP)**

• **OSAKA UNIVERSITY**
**Suita-shi**
**Osaka 565-0871 (JP)**

(72) Inventors:
• **YAMADA Asuka**
**Tokyo 110-0016 (JP)**
• **KITANO Shiro**
**Tokyo 110-0016 (JP)**
• **MATSUSAKI Michiya**
**Suita-shi, Osaka 565-0871 (JP)**

(74) Representative: **TBK**
**Bavariaring 4-6**
**80336 München (DE)**

(54) **PRODUCTION METHOD OF FRAGMENTED EXTRACELLULAR MATRIX COMPONENT**

(57)      One aspect of the present invention relates to a production method of a fragmented extracellular matrix component, the method including: a step of neutralizing a solution in which an extracellular matrix component is dissolved; a step of obtaining a mass containing the extracellular matrix component by removing the solvent through freeze-drying treatment after the neutralization step; a step of obtaining a liquid containing the fragmented extracellular matrix component by dispersing the mass in a solution containing a polar organic solvent and fragmenting the extracellular matrix component in the solution; and a step of removing a liquid component from the liquid containing the fragmented extracellular matrix component.

## Description

## Technical Field

The present invention relates to a production method of a fragmented extracellular matrix component. The present invention also relates to a fragmented extracellular matrix component and a production method of a cell structure using the same.

## Background Art

Technology for producing a cell structure imitating a biological tissue in vitro is known. As a cell culture method for obtaining a cell structure, cells are generally cultured in the presence of extracellular matrix components such as a collagen.

In addition, a method of using fragmented extracellular matrix components is known as a method for obtaining a cell structure in which the concentration of extracellular matrix components such as a collagen is increased to a concentration close to that of a biological tissue. For example, Patent Literature 1 discloses an agent for forming a three-dimensional tissue body containing a fragmented collagen, in which the fragmented collagen has an average length of 100 nm to 200 $\mu$m and an average diameter of 50 nm to 30 $\mu$m.

## Citation List

## Patent Literature

[Patent Literature 1] PCT International Publication No. WO2018/143286

## Summary of Invention

## Technical Problem

A fragmented extracellular matrix component in the related art has been obtained by dispersing freeze-dried extracellular matrix components such as a collagen in a buffer solution and then homogenizing the resultant with a homogenizer or the like. On the other hand, when the obtained fragmented extracellular matrix component is dried again through freeze-drying or the like for preservation, there is a problem that it cannot be easily re-dispersed in an aqueous solution. For this reason, in the related art, it is necessary to prepare a fragmented extracellular matrix component when in use. In addition, during preparation when in use, there is a problem that a part of the extracellular matrix component such as a collagen is dissolved in the buffer solution, resulting in a decrease in yield.

An object of the present invention is to provide a production method of a fragmented extracellular matrix component which can obtain a fragmented extracellular matrix component having excellent redispersibility even when stored in a dry state and has an improved yield as compared to the related art. Another object of the present invention is to provide a fragmented extracellular matrix component having excellent redispersibility even when stored in a dry state and a production method of a cell structure in which the fragmented extracellular matrix component is used.

## Solution to Problem

The present invention relates to, for example, each of the following inventions.

[1] A production method of a fragmented extracellular matrix component, including: a step of neutralizing a solution in which an extracellular matrix component is dissolved; a step of obtaining a mass containing the extracellular matrix component by removing the solvent through freeze-drying treatment after the above-described neutralization step; a step of obtaining a liquid containing the fragmented extracellular matrix component by dispersing the above-described mass in a solution containing a polar organic solvent and fragmenting the extracellular matrix component in the solution; and a step of removing a liquid component from the liquid containing the above-described fragmented extracellular matrix component.

[2] The production method according to [1], further including: a step of replacing the liquid component in the above-described liquid obtained in the step of obtaining the above-described liquid with water after the above-described step of obtaining the liquid and before the above-described step of removing the liquid component, in which, in the above-described step of removing the liquid component, the liquid component is removed from the liquid containing the fragmented extracellular matrix component through freeze-drying treatment.

[3] The production method according to [2], further including: a step of subjecting the liquid containing the above-described fragmented extracellular matrix component to ultrasonic crushing treatment after the above-described step of performing replacement with the water and before the above-described step of removing the liquid component.

[4] The production method according to any one of [1] to [3], further including: a step of gelling the above-described neutralized solution after the above-described neutralization step and before the above-described step of obtaining the mass.

[5] The production method according to [4], in which the above-described mass is a porous body.

[6] The production method according to any one of [1] to [5], in which the above-described fragmentation includes defibration of an extracellular matrix component.

[7] The production method according to any one of [1] to [6], in which the concentration of the above-described polar organic solvent in the solution containing the above-described polar organic solvent is 20% v/v to 100% v/v.

[8] The production method according to any one of [1] to [7], in which the above-described polar organic solvent includes ethanol.

[9] The production method according to any one of [1] to [8], further including: a step of dispersing a solid matter obtained in the above-described step of removing the liquid component in an aqueous medium.

[10] The production method according to any one of [1] to [9], in which the above-described extracellular matrix component is a collagen.

[11] The production method according to [10], in which the above-described fragmented extracellular matrix component has a triple helix structure specific to a collagen.

[12] A production method of a cell structure, including: a step of mixing cells with the fragmented extracellular matrix component obtained through the production method according to any one of [1] to [11]; and a step of incubating the mixture obtained in the above-described mixing step.

[13] A dried product of fragmented extracellular matrix components, in which fibrous extracellular matrix components having an average diameter of 0.08 $\mu$m to 2.0 $\mu$m are stacked.

[14] The dried product of fragmented extracellular matrix components according to [13], in which, in an observation image acquired by preparing a slice specimen and observing a rectangular region of the above-described slice specimen with a long side of 1820 $\mu$m and a short side 1365 $\mu$m with a microscope, an area of a region where the extracellular matrix components are present in the area of the above-described observation image is 40% to 100%.

[15] The dried product of fragmented extracellular matrix components according to [13] or [14], in which the above-described fragmented extracellular matrix components are fragmented collagens.

[16] A fragmented extracellular matrix component having a peak within a wavelength range of 400 nm to 550 nm as measured by fluorescence intensity using 1,8-anilinonaphthalene sulfonic acid (ANS).

## Advantageous Effects of Invention

According to the present invention, it is possible to provide a production method of a fragmented extracellular matrix component which can obtain a fragmented extracellular matrix component having better redispersibility even when stored in a dry state and has an improved yield as compared to the related art. According to the present invention, it is also possible to provide a fragmented extracellular matrix component having better redispersibility even when stored in a dry state and a production method of a cell structure in which the fragmented extracellular matrix component is used.

## Brief Description of Drawings

FIG. 1 illustrates optical microscope photographs of fragmented collagens re-suspended in ultrapure water. FIG. 1(A) is a fragmented collagen obtained in Production Example 1. FIG. 1(B) is a fragmented collagen obtained in Comparative Production Example 1.

FIG. 2 is a CD spectrum of the fragmented collagen obtained in Production Example 1.

FIG. 3 illustrates photographs of hematoxylin-eosin (HE)-stained fragmented collagens. FIGS. 3(A) and 3(C) are photographs of the fragmented collagen obtained in Comparative Production Example 1 respectively imaged at 10× and 40× magnification. FIGS. 3(B) and 3(D) are photographs of the fragmented collagen obtained in Production Example 1 respectively imaged at 10× and 40× magnification.

FIG. 4 illustrates photographs of fragmented collagens imaged with a scanning electron microscope. FIGS. 4(A) and 4(B) are photographs of the fragmented collagen obtained in Comparative Production Example 1 respectively imaged at 2000× and 3300× magnification. FIGS. 4(C) and 4(D) are photographs of the fragmented collagen obtained in Production Example 1 respectively imaged at 2000× and 3300× magnification.

FIG. 5 illustrates optical microscope photographs of fragmented collagens re-suspended in ultrapure water. FIG.

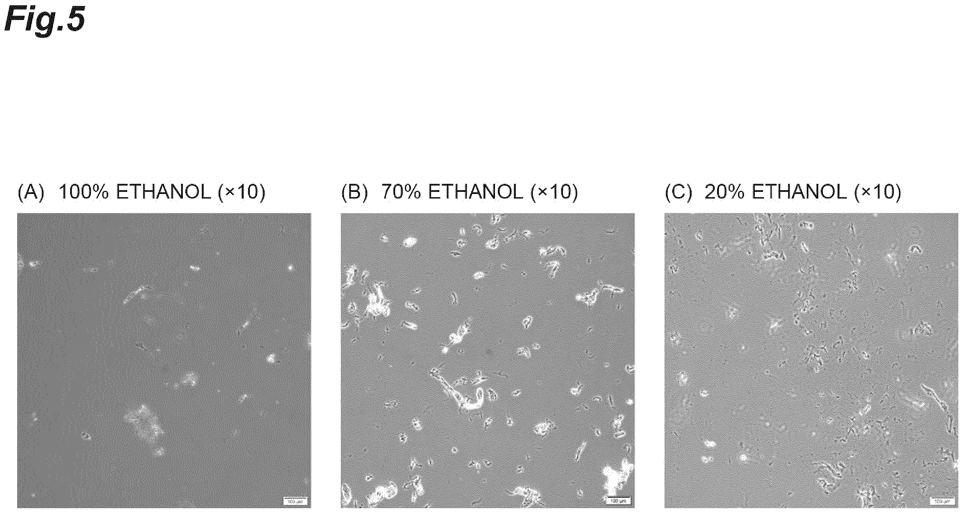

5(A) is a fragmented collagen obtained in Production Example 1. FIG. 5(B) is a fragmented collagen obtained in Production Example 2-3. FIG. 5(C) is a fragmented collagen obtained in Production Example 2-1.

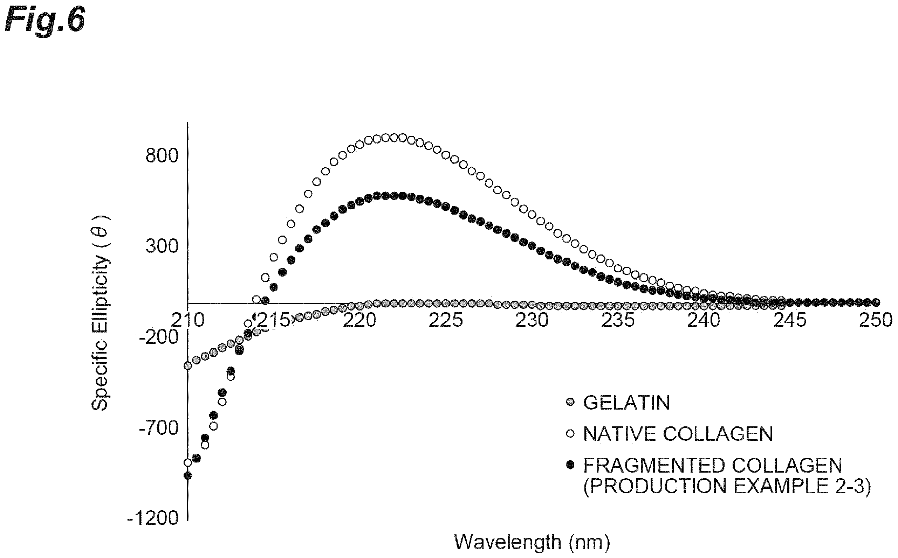

FIG. 6 is a CD spectrum of the fragmented collagen obtained in Production Example 2-3.

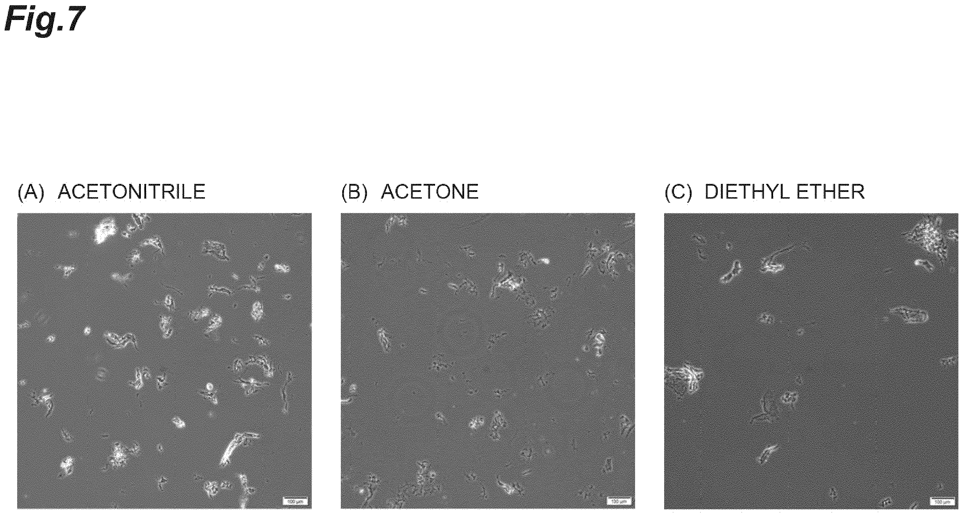

FIG. 7 illustrates optical microscope photographs of fragmented collagens re-suspended in ultrapure water. FIG. 7(A) is a fragmented collagen obtained in Production Example 3-1. FIG. 7(B) is a fragmented collagen obtained in Production Example 3-2. FIG. 7(C) is a fragmented collagen obtained in Production Example 3-3.

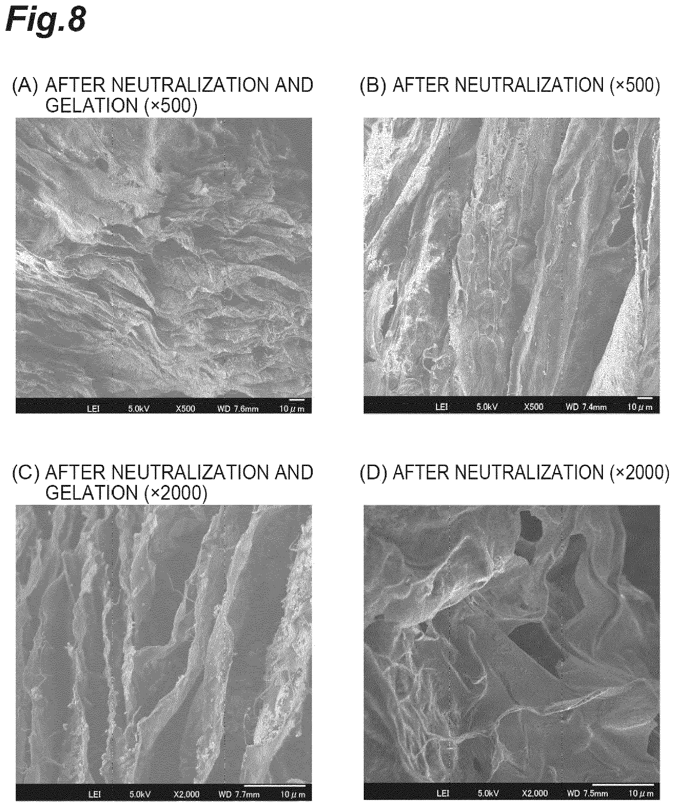

FIG. 8 illustrates photographs of collagens imaged with a scanning electron microscope after neutralization and after neutralization and gelation. FIGS. 8(A) and 8(C) are photographs of a collagen respectively imaged at 500× and 2000× magnification after neutralization and gelation. FIGS. 8(B) and 8(D) are photographs of a collagen respectively imaged at 500× and 2000× magnification after neutralization.

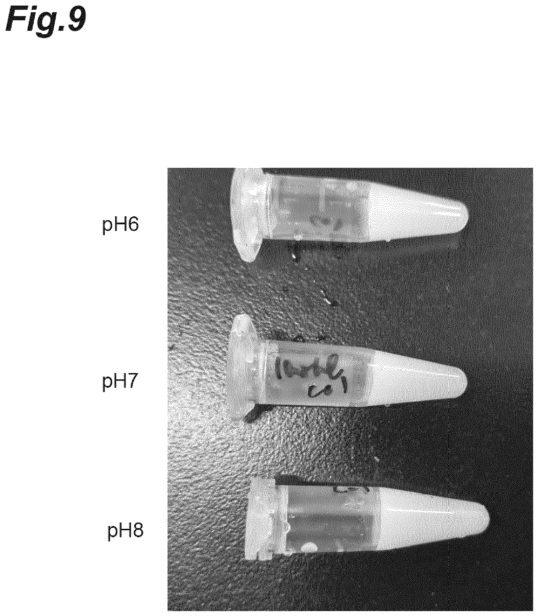

FIG. 9 is a photograph of gelled collagen solutions.

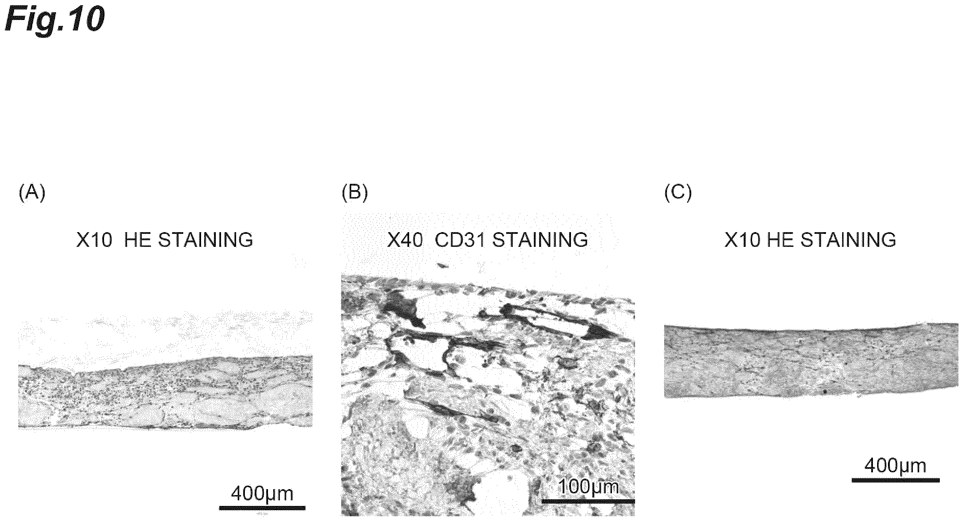

FIG. 10 illustrates photographs of cell structures stained with hematoxylin and eosin (HE) (FIGS. 10(A) and 10(C)) and immunostained with CD31 (FIG. 10(B)). FIGS. 10(A) and 10(B) are cell structures produced using the fragmented collagen obtained in Production Example 1. FIG. 10(C) is a cell structure produced using the fragmented collagen obtained in Comparative Production Example 1.

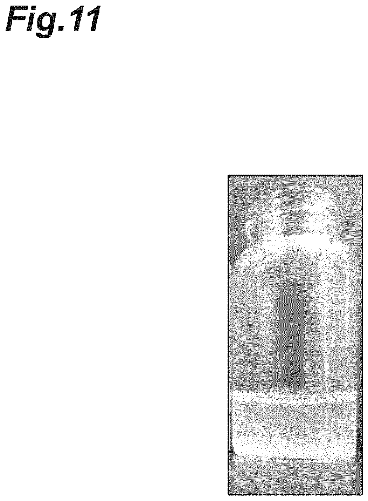

FIG. 11 is a photograph of a fragmented collagen dispersed through a pipetting operation.

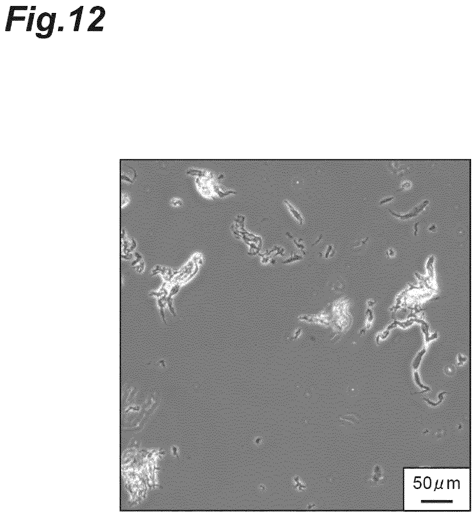

FIG. 12 is a phase-contrast micrograph of a re-dispersed fragmented collagen.

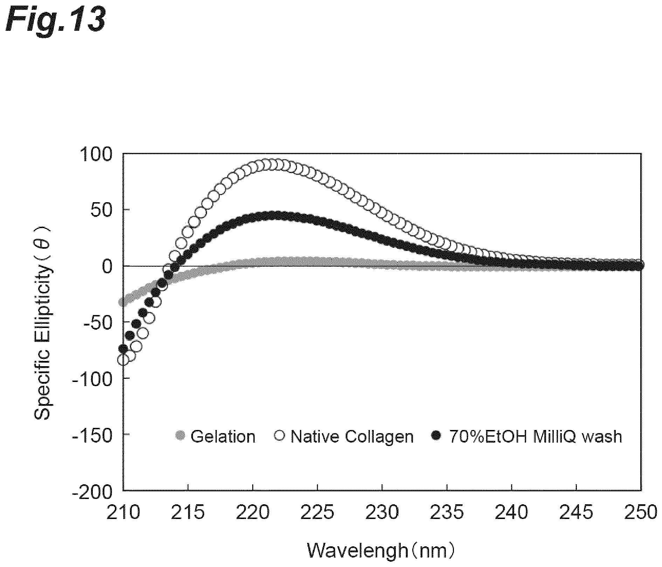

FIG. 13 illustrates CD spectra of gelatin, a raw material collagen (Native Collagen), and a fragmented collagen obtained through a method of Production Example 5-4.

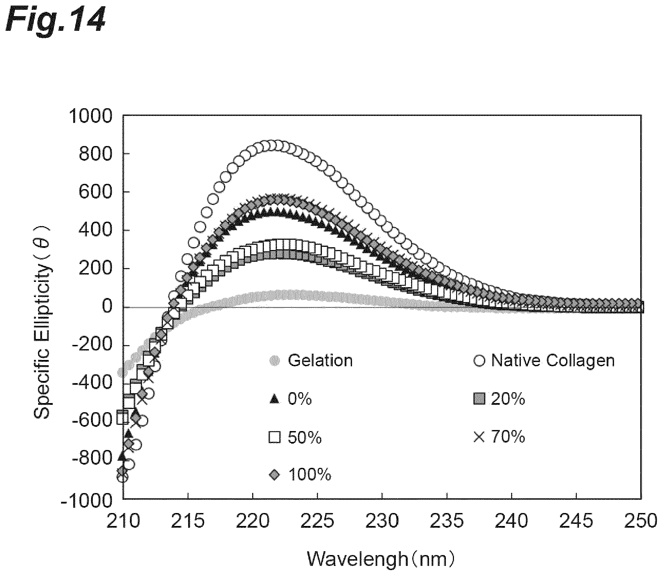

FIG. 14 illustrates CD spectra of gelatin, a raw material collagen (Native Collagen), and fragmented collagens obtained through methods of Production Examples 5-1 to 5-5.

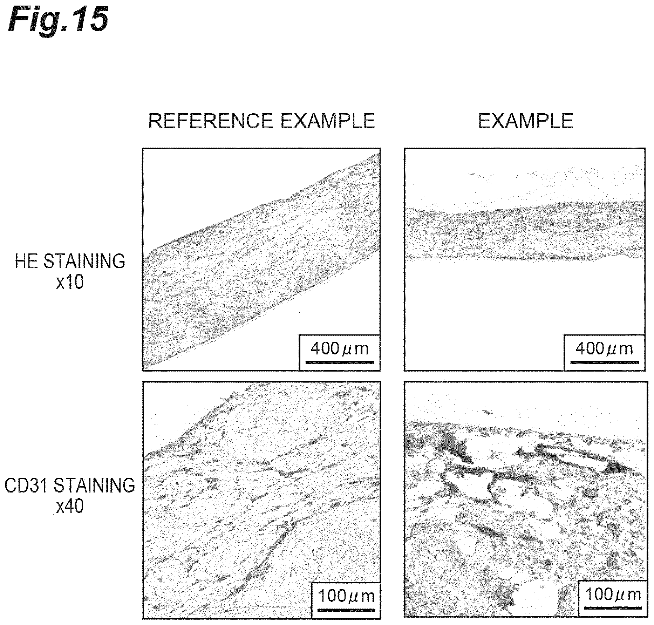

FIG. 15 illustrates photographs showing slice images of hematoxylin-eosin (HE)-stained and CD31-stained cell structures produced using fragmented collagens.

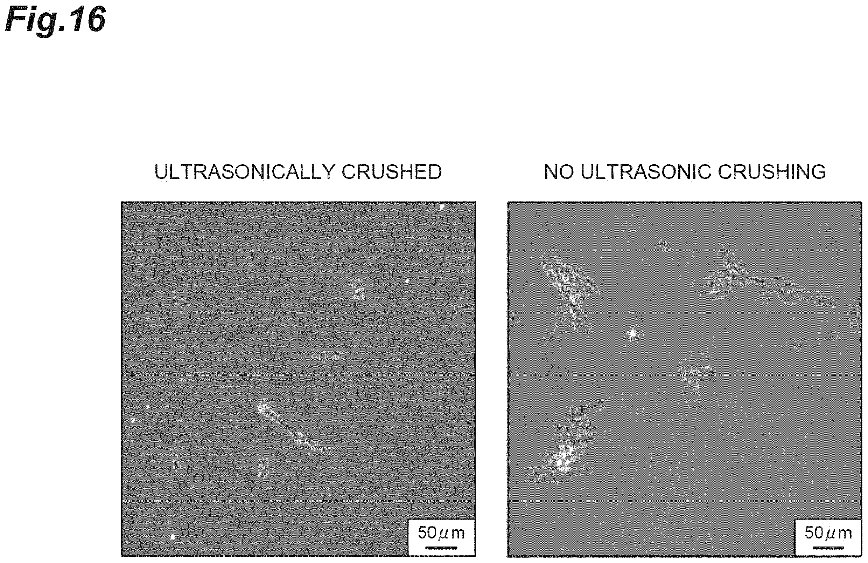

FIG. 16 illustrates phase-contrast micrographs showing evaluation results of the influence of the presence or absence of ultrasonic defibration on a fragmented collagen.

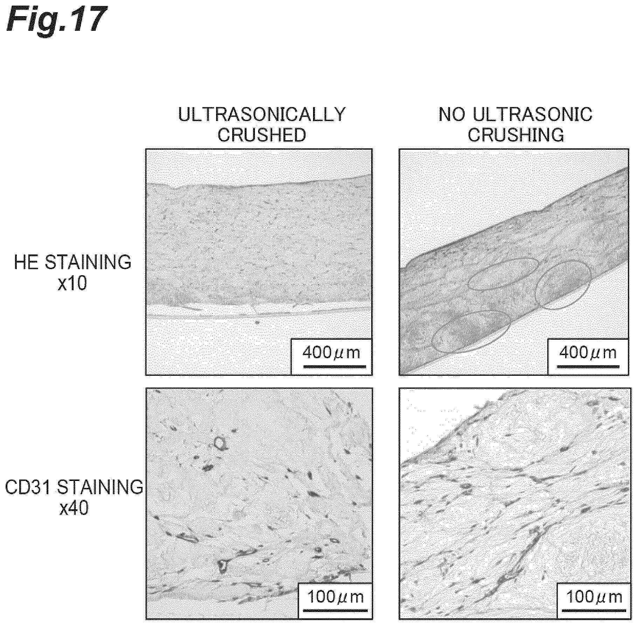

FIG. 17 illustrates photographs showing slice images of cell structures showing evaluation results of the influence of the presence or absence of ultrasonic defibration on fragmented collagens.

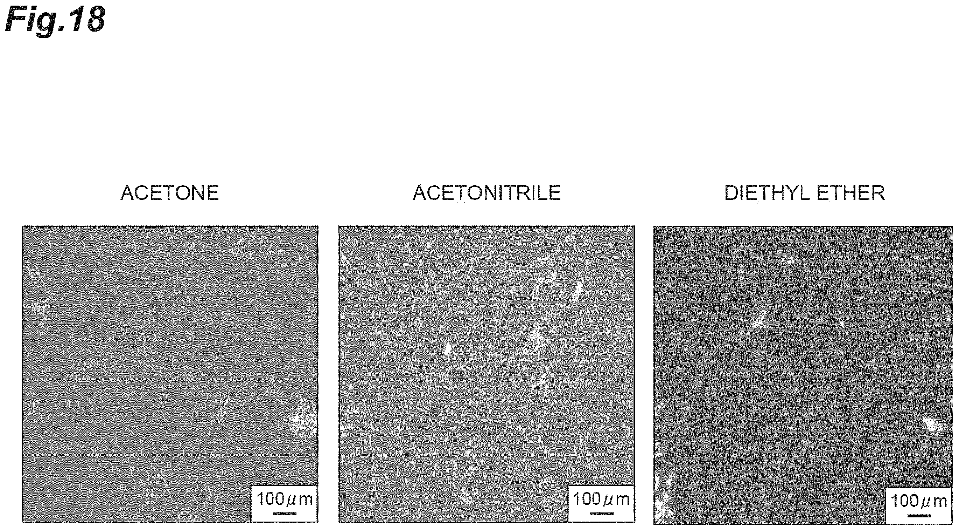

FIG. 18 illustrates micrographs showing observation results of fragmented collagens produced using acetone, acetonitrile, and diethyl ether as polar organic solvents.

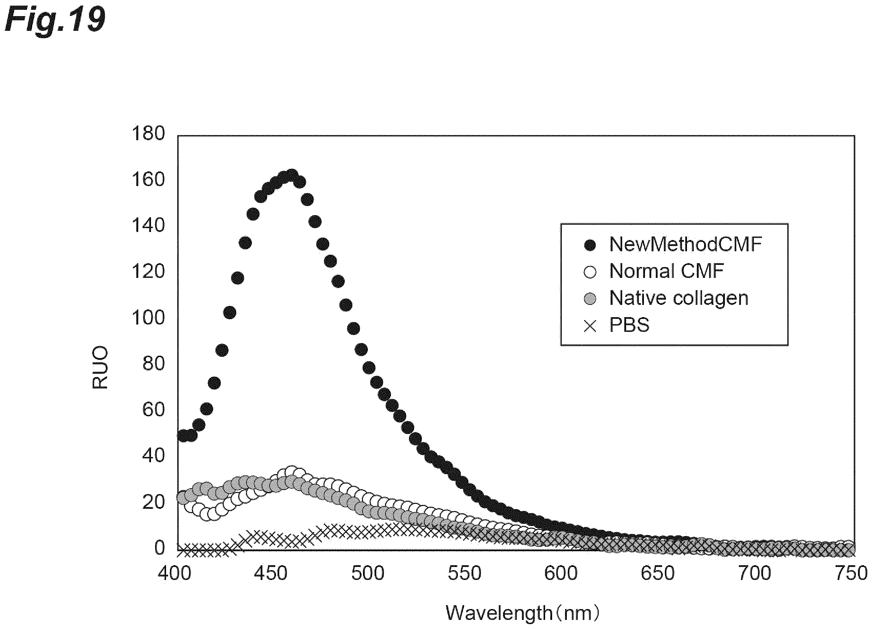

FIG. 19 is a graph showing evaluation results of hydrophobicity.

## Description of Embodiments

Hereinafter, embodiments of the present invention will be described in detail. However, the present invention is not limited to the following embodiments.

[Production method of fragmented extracellular matrix component]

A production method of a fragmented extracellular matrix component according to the present embodiment includes: a step of neutralizing a solution in which an extracellular matrix component is dissolved (neutralization step); a step of obtaining a mass containing the extracellular matrix component by removing the solvent through freeze-drying treatment after the neutralization step (solvent removal step); a step of obtaining a liquid containing the fragmented extracellular matrix component by dispersing the mass in a solution containing a polar organic solvent and fragmenting the extracellular matrix component in the solution (fragmentation step); and a step of removing a liquid component from the liquid containing the fragmented extracellular matrix component (drying step).

The production method of a fragmented extracellular matrix component according to the present embodiment may further include: in addition to the above-described steps, a step of gelling the neutralized solution after the neutralization step and before the solvent removal step (gelation step).

The production method of a fragmented extracellular matrix component according to the present embodiment may further include: in addition to the above-described steps, a step of replacing the liquid component in the liquid obtained in the fragmentation step with water (replacement step) after the fragmentation step and before the drying step.

The production method of a fragmented extracellular matrix component according to the present embodiment may further include: in addition to the above-described steps, a step of subjecting the liquid containing the fragmented extracellular matrix component to ultrasonic crushing treatment (ultrasonic crushing step) after the replacement step and before the drying step.

The production method of a fragmented extracellular matrix component according to the present embodiment may further include: in addition to the above-described steps, a step of dispersing a solid matter obtained in the drying step in an aqueous medium (washing step).

(Neutralization step)

The neutralization step is a step of neutralizing the solution in which the extracellular matrix component is dissolved. Since neutralization changes a structure of the extracellular matrix, by performing it together with subsequent steps, a fragmented extracellular matrix component having better redispersibility can be obtained even when stored in a dry state.

The "extracellular matrix component" in the present specification is an extracellular matrix molecule aggregate formed by a plurality of extracellular matrix molecules (also simply referred to as an "extracellular matrix"). The extracellular matrix means a substance existing outside cells in an organism. An arbitrary substance can be used as the extracellular matrix as long as this does not adversely affect growth of cells and formation of cell aggregates. Specific examples of extracellular matrixes include collagens, elastin, proteoglycans, fibronectin, hyaluronic acid, laminin, vitronectin, tenascin, entactin, and fibrillin, but the present invention is not limited thereto. Extracellular matrix components may include a single extracellular matrix or may include a combination of two or more extracellular matrixes.

Extracellular matrixes may be modified extracellular matrixes or variants of extracellular matrixes or may be polypeptides such as chemically synthesized peptides as long as these do not adversely affect the growth of cells and the formation of cell aggregates. Extracellular matrixes may have a repetition of a sequence represented by Gly-X-Y which is characteristic to a collagen. Here, Gly represents a glycine residue, and X and Y each independently represent an arbitrary amino acid residue. A plurality of Gly-X-Y's may be the same as or different from each other. If extracellular matrixes have a repetition of a sequence represented by Gly-X-Y, the degree of binding to a molecular chain arrangement is small. Therefore, for example, the function as a scaffold material during culturing cells is further improved. In the extracellular matrixes having the repetition of the sequence represented by Gly-X-Y, the proportion of the sequence represented by Gly-X-Y in the whole amino acid sequence may be 80% or higher and preferably 95% or higher. In addition, the extracellular matrixes may be polypeptides having an RGD sequence. The RGD sequence is a sequence represented by Arg-Gly-Asp (arginine residue-glycine residue-aspartic acid residue). If the extracellular matrixes have an RGD sequence, cell adhesion is further promoted, and therefore the extracellular matrixes are even more suitable as scaffold materials during culturing cells, for example. Examples of extracellular matrixes having the sequence represented by Gly-X-Y and the RGD sequence include collagens, fibronectin, vitronectin, laminin, and cadherin.

Examples of collagens include a fibrous collagen and a non-fibrous collagen. A fibrous collagen means a collagen that is a main component of collagen fibers, and specific examples thereof include a type I collagen, a type II collagen, and a type III collagen. Examples of non-fibrous collagens include a fibrous type IV collagen.

Examples of proteoglycans include, but are not limited to, chondroitin sulfate proteoglycans, heparan sulfate proteoglycans, keratan sulfate proteoglycans, and dermatan sulfate proteoglycans.

An extracellular matrix component may contain at least one selected from the group consisting of a collagen, laminin, and fibronectin and preferably contains a collagen. A collagen is preferably a fibrous collagen and more preferably a type I collagen. A commercially available collagen may be used as a fibrous collagen, and specific examples thereof include porcine skin-derived type I collagen manufactured by NH Foods Ltd.

An extracellular matrix component may be derived from animals. Examples of animal species from which extracellular matrix components are derived include, but are not limited to, humans, pigs, and cattle. A component derived from one type of animal may be used as an extracellular matrix component, or components derived from plural kinds of animals may be used in combination.

A solvent for the solution in which an extracellular matrix component is dissolved is not particularly limited as long as it can dissolve the extracellular matrix component. Specific examples of solvents include water, a buffer solution (for example, a phosphate-buffered physiological saline (PBS) and a tris-hydrochloric acid (Tris-HCl) buffer solution).

The concentration of an extracellular matrix component in a solution in which the extracellular matrix component is dissolved is not particularly limited, but may be, for example, 1 mg/mL to 50 mg/mL.

Since the solution in which the extracellular matrix component is dissolved is usually acidic, the neutralization step can be performed by, for example, adding an alkaline solution to the solution in which the extracellular matrix component is dissolved. The alkaline solution is not particularly limited, but for example, a solution in which an alkali metal hydroxide, an alkaline earth metal hydroxide, or the like is dissolved in water can be suitably used. More specific examples alkaline solutions include a potassium hydroxide (KOH) aqueous solution and a sodium hydroxide (NaOH) aqueous solution.

In a case where the solution in which the extracellular matrix component is dissolved is alkaline, the neutralization step can be carried out by, for example, adding an acidic solution to the solution in which the extracellular matrix component is dissolved. The acidic solution is not particularly limited, but examples thereof include a hydrochloric acid solution, a sulfuric acid solution, an acetic acid solution, and a carbonic acid solution.

Neutralization may be carried out so that a structural change of the extracellular matrix occurs, and is not necessarily carried out so that the pH of the solution after neutralization becomes 7. Specifically, the pH of the solution after neutralization may be, for example, within a range of 6 to 8, preferably within a range of 6.5 to 7.5, more preferably within a range of 6.9 to 7.1, and still more preferably within a range of 6.95 to 7.05.

(Gelation step)

The gelation step is a step of gelling the neutralized solution after the neutralization step and before the solvent removal step. The gelation step may be carried out as necessary.

The gelation step can be carried out, for example, by heating a neutralized solution to gel it. The temperature during heating and the heating time can be appropriately set depending on the type, concentration, and the like of an extracellular matrix component, but for example, the temperature during heating can be set to 25°C to 45°C and the heating time can be set to 1 to 3 hours.

By carrying out the gelation step, it is possible to confirm whether the solution has been uniformly neutralized in the neutralization step by visually determining whether the formed gel is uniform. In addition, by carrying out the gelation step, the mass containing the extracellular matrix component obtained in the solvent removal step becomes a porous body, and fragmentation in the fragmentation step can be carried out more efficiently.

(Solvent removal step)

The solvent removal step is a step of removing the solvent through freeze-drying treatment after the neutralization step (or after the gelation step in a case where the gelation step is included) to obtain a mass containing the extracellular matrix component.

The freeze-drying treatment of a solution after neutralization or gel after gelation can be carried out according to a usual method. The solvent is removed through the solvent removal step, and a mass (solid) containing the extracellular matrix component is obtained. Removal of a solvent does not mean that there is no solvent at all attached to a mass (solid) containing an extracellular matrix component, but means that no solvent is attached thereto to a degree that it can be reached by common sense through usual freeze-drying treatment.

(Fragmentation step)

The fragmentation step is a step of obtaining a liquid containing the fragmented extracellular matrix component by dispersing the mass obtained in the solvent removal step in a solution containing a polar organic solvent and fragmenting the extracellular matrix component in the solution.

By performing fragmentation in the solution containing the polar organic solvent, a fragmented extracellular matrix component having better redispersibility can be obtained even when stored in a dry state and the extracellular matrix component can be suppressed from being dissolved in a solution, resulting in a higher yield compared to a conventional method.

Furthermore, by performing fragmentation in the solution containing the polar organic solvent, it is possible to obtain a fragmented extracellular matrix component that does not easily dissolve even if the liquid component is replaced with water in a subsequent step.

The polar organic solvent is not particularly limited as long as it has polarity, and examples thereof include alcohols such as methanol, ethanol, n-propanol, and isopropanol, acetone, acetonitrile, and diethyl ether.

The concentration of the polar organic solvent in the solution containing the polar organic solvent is not particularly limited, but is preferably 20% v/v to 100% v/v from the viewpoint of more significantly exhibiting the above-described effect. The concentration of the polar organic solvent in the solution containing the polar organic solvent may be, for example, 20% v/v to 50% v/v, 20% v/v to 40% v/v, or may be 20% v/v to 30% v/v. The concentration of the polar organic solvent in the solution containing the polar organic solvent may be 50% v/v to 100% v/v, 50% v/v to 90% v/v, or 50% v/v to 80% v/v or may be 60% v/v to 100% v/v, 60% v/v to 90% v/v, or 60% v/v to 80% v/v, for example.

In the present specification, the "fragmentation" means that an aggregate of extracellular matrix molecules is made smaller in size. The fragmentation may be performed under the conditions of cleaving the bond within extracellular matrix molecules or may be performed under the conditions of not cleaving the bond within extracellular matrix molecules. Unlike enzymatic treatment, an extracellular matrix fragmented through application of physical force usually does not change in molecular structure from before the fragmentation (the molecular structure is maintained). The fragmented extracellular matrix components may include defibrated extracellular matrix components in which the above-described extracellular matrix component is defibrated through application of physical force. Defibration is an aspect of fragmentation and is performed under the conditions of, for example, not cleaving the bond within extracellular matrix molecules.

In the production method of a fragmented extracellular matrix component according to the present embodiment, the fragmentation step may be configured as a step of obtaining a liquid containing the defibrated extracellular matrix component by dispersing the mass obtained in the solvent removal step in a solution containing a polar organic solvent and defibrating the extracellular matrix component in the solution (defibration step).

The method for fragmenting an extracellular matrix component is not particularly limited but may be performed through application of physical force. As the method for fragmenting extracellular matrix components, extracellular matrix components may be fragmented through application of physical force with homogenizers such as an ultrasonic homogenizer, a stirring homogenizer, and a high-pressure homogenizer, for example. Millimeter-sized and nanometer-sized fragmented extracellular matrix components can also be obtained by adjusting the time, the number of times of homogenizing or the like.

Examples of more specific conditions for fragmenting an extracellular matrix component include conditions for performing a treatment at 20,000 rpm to 30,000 rpm for 3 to 10 minutes using a homogenizer (manufactured by AS ONE Corporation, VH-10) or conditions under which physical force corresponding thereto can be applied.

Fragmented extracellular matrix components may include at least some defibrated extracellular matrix components. In addition, fragmented extracellular matrix components may consist of only defibrated extracellular matrix components. That is, fragmented extracellular matrix components may be defibrated extracellular matrix components. Defibrated extracellular matrix components preferably include a defibrated collagen. A defibrated collagen preferably maintains a triple helix structure derived from a collagen. A defibrated collagen may be one partially maintaining a triple helix structure derived from a collagen.

Examples of shapes of fragmented extracellular matrix components include a fibrous shape. The fibrous shape means a shape composed of a filamentous extracellular matrix component or a shape composed of cross-linked filamentous extracellular matrix components between molecules. At least some fragmented extracellular matrix components may be fibrous. Fibrous extracellular matrix components include, for example, fine filamentous materials (fine fibers) formed by aggregating a plurality of filamentous extracellular matrix molecules, a filamentous material formed by further aggregating fine fibers, and one obtained by defibrating these filamentous materials. An RGD sequence is preserved without disruption in a fibrous extracellular matrix component.

The average length of fragmented extracellular matrix components may be 100 nm to 400 $\mu$m and 100 nm to 200 $\mu$m. In one embodiment, the average length of fragmented extracellular matrix components may be 5 $\mu$m to 400 $\mu$m, 10 $\mu$m to 400 $\mu$m, 22 $\mu$m to 400 $\mu$m, or 100 $\mu$m to 400 $\mu$m. In another embodiment, the average length of pieces of fragmented extracellular matrix components may be less than or equal to 100 $\mu$m, less than or equal to 50 $\mu$m, less than or equal to 30 $\mu$m, less than or equal to 15 $\mu$m, less than or equal to 10 $\mu$m, or less than or equal to 1 $\mu$m, and greater than or equal to 100 nm. Of all the fragmented extracellular matrix components, the average length of most of the fragmented extracellular matrix components is preferably within the above-described numerical ranges. Specifically, the average length of 95% of the fragmented extracellular matrix components of all the fragmented extracellular matrix components is preferably within the above-described numerical ranges.

The average diameter of fragmented extracellular matrix components may be 10 nm to 10 $\mu$m, 20 nm to 8 $\mu$m, 30 nm to 6 $\mu$m, or 50 nm to 4 $\mu$m.

The average length and the average diameter of fragmented extracellular matrix components can be obtained by measuring each fragmented extracellular matrix component using an optical microscope and performing image analysis. In the present specification, the "average length" means an average value of the lengths of the measured samples in the longitudinal direction and the "average diameter" means an average value of the lengths of the measured samples in the direction orthogonal to the longitudinal direction.

Through the fragmentation step, a liquid containing the fragmented extracellular matrix component can be obtained. The liquid includes, for example, a solution containing the polar organic solvent in addition to the fragmented extracellular matrix component.

(Replacement step)

The replacement step is a step of replacing the liquid component in the liquid obtained in the fragmentation step with water after the fragmentation step and before the drying step.

By replacing the liquid component in the liquid obtained in the fragmentation step with water before the drying step, a fragmented extracellular matrix component obtained through the drying step can be rapidly re-dispersed.

Examples of water include tap water, distilled water, and ultrapure water.

The method for replacing the liquid component in the liquid obtained in the fragmentation step with water is not particularly limited, but a usual method carried out during solvent replacement can be used. Examples of the replacement method with water include a method for centrifuging the liquid obtained in the fragmentation step, allowing the fragmented extracellular matrix component in the liquid to settle, subsequently removing the liquid component, and then adding water to the fragmented extracellular matrix component. The substitution of the liquid component with water does not mean that all liquid components present in the liquid are replaced with water, but means that a main solvent in the liquid is replaced with water, and a trace amount of liquid components other than water may remain after the replacement.

The liquid obtained through the replacement step includes water in addition to the fragmented extracellular matrix component.

(Ultrasonic crushing process)

The ultrasonic crushing step is a step of subjecting the liquid obtained in the fragmentation step to ultrasonic crushing treatment. The ultrasonic crushing step may be carried out as necessary. Through the ultrasonic crushing step, it is possible to obtain a fragmented extracellular matrix component which is more uniformly dispersed and less prone to aggregation when used for organization. The ultrasonic crushing treatment has advantages that the fragmented extracellular matrix component is less likely to be thermally denatured because it can be crushed while being cooled, and that a large-scale facility is not required.

The ultrasonic crushing step can be carried out using an ultrasonic crusher. As the ultrasonic crusher, for example, a fully automatic ultrasonic crusher such as BIORUPTOR II manufactured by BM Equipment Co., Ltd.

The ultrasonic crushing treatment may be carried out by repeating irradiation of the liquid obtained in the fragmentation step with an ultrasonic wave and cooling of the liquid. The ultrasonic crushing treatment may be carried out by, for example, repeating ultrasonic irradiation for 10 to 30 seconds and cooling for 20 to 40 seconds 50 to 150 times at an ultrasonic frequency of 20 kHz.

In the conventional method that do not involve fragmentation in a solution containing a polar organic solvent, a fragmented extracellular matrix component may dissolve when an ultrasonic crushing treatment is performed while cooling. For this reason, to obtain a finely fragmented extracellular matrix component through an ultrasonic crushing treatment, it is necessary to, for example, perform a thermal cross-linking treatment on the fragmented extracellular matrix component or a precursor thereof to prevent dissolution during ultrasonic crushing. However, the fragmented extracellular matrix component may be denatured by the thermal cross-linking treatment, and tissue obtained using the denatured fragmented extracellular matrix component may be significantly different from biological tissues. The fragmented extracellular matrix component obtained through fragmentation in a solution containing a polar organic solvent does not dissolve easily even if the liquid component is replaced with water. Therefore, the fragmented extracellular matrix component obtained through the above-described fragmentation step and the above-described replacement step can be subjected to an ultrasonic crushing treatment without undergoing a treatment that can denature the fragmented extracellular matrix component. Accordingly, it is possible to obtain a fragmented extracellular matrix component which is more uniformly dispersed and less prone to aggregation when used for organization.

(Drying step)

The drying step is a step of removing a liquid component from the liquid containing the fragmented extracellular matrix component obtained in the fragmentation step.

Removal of the liquid component can be carried out through, for example, air drying, freeze-drying treatment, vacuum drying treatment, and vacuum freeze-drying treatment. Removal of the liquid component may be carried out through air drying or freeze-drying treatment. It is preferable to remove the liquid component through air drying because a fragmented extracellular matrix component having better redispersibility is likely to be obtained even when stored in a dry state.

The liquid component is removed through the drying step, and a solid matter containing a dried product of fragmented extracellular matrix components is obtained. Removal of the liquid component does not mean that there is no liquid component at all attached to a solid matter containing a dried product of fragmented extracellular matrix components, but means that there is no liquid component to a degree that can be reasonably reached through the above-described usual drying technique.

(Washing step)

The washing step is a step of dispersing the solid matter obtained in the drying step in an aqueous medium. The washing step may be carried out as necessary. Through the washing step, unnecessary components (for example, salts) are removed from a solid matter containing fragmented extracellular matrix components, and fragmented extracellular matrix components with higher purity can be obtained.

The aqueous medium used in the washing step is preferably one in which fragmented extracellular matrix components can be dispersed and the above-described unnecessary components can be dissolved. Specific examples of aqueous media include water, a phosphate-buffered physiological saline (PBS), and a tris buffer solution.

Through the production method of a fragmented extracellular matrix component according to the present embodiment, a dried product of fragmented extracellular matrix components can be obtained at a higher yield than a conventional method. The obtained dried product of the fragmented extracellular matrix components has better redispersibility and can be easily re-dispersed by, for example, adding an arbitrary solvent to perform a pipetting operation.

A production method of a fragmented extracellular matrix component according to one embodiment may be a method including: a neutralization step, a solvent removal step, a fragmentation step, a replacement step, and a drying step, in which the drying step is a step of removing a liquid component from a liquid obtained in the replacement step through freeze-drying treatment. A dried product of the fragmented extracellular matrix components obtained through the method further including the above-described replacement step can be more rapidly re-dispersed. For example, in a method that does not include the above-described steps, it may take about 3 to 5 minutes to perform redispersion by adding a solvent to perform a pipetting operation. The dried product of the fragmented extracellular matrix components obtained through the method including the above-described neutralization step, the above-described solvent removal step, the above-described fragmentation step, the above-described replacement step, and the above-described drying step can be re-dispersed in a shorter time (for example, for about 1 minute).

The following are considered as reasons why a fragmented extracellular matrix component of one embodiment obtained through a method including the method including the above-described neutralization step, the above-described solvent removal step, the above-described fragmentation step, the above-described replacement step, and the above-described drying step can be re-dispersed more rapidly. Since conventional fragmented extracellular matrix components are highly hydrophilic even once defibrated, the fragmented extracellular matrix components are entangled with each other again during the freeze-drying stage. On the other hand, the fragmented extracellular matrix components according to the above-described embodiments have high hydrophobicity and are re-entangled with each other through water, and therefore, can be freeze-dried in an easily dispersible state. As a result, it is thought that the fragmented extracellular matrix components can be more rapidly re-dispersed. It is thought that the fragmented extracellular matrix components acquire hydrophobicity in the process of replacing the fragmented extracellular matrix components with water and then performing freeze-drying treatment. However, the reason why the fragmented extracellular matrix components can be more promptly re-dispersed is not limited to the above.

[Dried product of fragmented extracellular matrix components]

A dried product of the fragmented extracellular matrix components according to the present embodiment can be typically obtained through the above-described production method of a fragmented extracellular matrix component according to the present embodiment.

In a dried product of fragmented extracellular matrix components according to the present embodiment, fibrous extracellular matrix components having an average diameter of 0.08 μm to 2.0 μm may be stacked. Having such a configuration provides excellent redispersibility. The average diameter is preferably 0.1 μm to 1.5 μm, more preferably 0.3 μm to 1.0 μm, and still more preferably 0.5 μm to 0.7 μm.

In the dried product of fragmented extracellular matrix components according to the present embodiment, in an observation image acquired by preparing a slice specimen and observing a rectangular region of the slice specimen with a long side of 1820 μm and a short side 1365 μm using a microscope, an area of a region where the extracellular matrix components are present in the area of the observation image may be 40% to 100%. Having such a configuration provides excellent redispersibility. The above-described proportion may be 40% to 95%, 40% to 90%, 40% to 85%, 40% to 80%, or 40% to 75%, may be 45% to 100%, 45% to 95%, 45% to 90%, 45% to 85%, 45% to 80%, or 45% to 75%, may be 50% to 100%, 50% to 95%, 50% to 90%, 50% to 85%, 50% to 80%, or 50% to 75%, may be 55% to 100%, 55% to 95%, 55% to 90%, 55% to 85%, 55% to 80%, or 55% to 75%, may be 60% to 100%, 60% to 95%, 60% to 90%, 60% to 85%, 60% to 80%, or 60% to 75%, or may be 65% to 100%, 65% to 95%, 65% to 90%, 65% to 85%, 65% to 80%, or 65% to 75%.

The fragmented extracellular matrix component according to the present embodiment has a peak within a wavelength range of 400 nm to 550 nm as measured by fluorescence intensity using 1,8-anilinonaphthalene sulfonic acid (ANS). The fragmented extracellular matrix component can be suitably produced through the above-described production method of a fragmented extracellular matrix component according to the present embodiment. The fragmented extracellular matrix component according to the present embodiment may have a peak within a wavelength range of 420 nm to 500 nm or 425 nm to 475 nm as measured by fluorescence intensity using ANS.

ANS is a hydrophobic probe. It can be determined that the fragmented extracellular matrix component has hydrophobicity in a case where this has a peak within a wavelength range of 400 nm to 550 nm as measured by fluorescence intensity using ANS.

A measurement sample for fluorescence intensity is prepared through the following method. A fragmented extracellular matrix component to be measured is added to phosphate-buffered physiological saline (1× PBS) to a concentration of 5 mg/mL. The solution to which the fragmented extracellular matrix component is added is stored in a refrigerator for 4 days to dissolve the fragmented extracellular matrix. This is used as a fragmented extracellular matrix solution. magnesium(II) 8-anilino-1-naphthalenesulfonate (ANS-8-Mg) is dissolved in phosphate-buffered physiological saline (1× PBS) to a concentration of 20 μM. This is used as an ANS-Mg aqueous solution. 200 μL of the ANS-Mg aqueous solution is added to 500 μL of the fragmented extracellular matrix solution to cause a reaction under a light shielding condition at room temperature (25°C) for 1 hour. The obtained reaction solution is used as a measurement sample.

Fluorescence intensity measurement is carried out under the conditions shown below.

Measurement instrument: Spectrophotometer (for example, NanoDrop™ 3300 Fluorometer (manufactured by Thermo Fischer Scientific Inc.)
Excitation wavelength: 380 nm
Measurement wavelength: 400 to 750 nm

Since the fragmented extracellular matrix component according to the present embodiment has a peak within a wavelength range of 400 nm to 550 nm as measured by fluorescence intensity using 1,8-anilinonaphthalene sulfonic acid (ANS), this can be rapidly re-dispersed.
The fluorescence intensity of the fragmented extracellular matrix component measured using ANS may be more than twice the fluorescence intensity of an extracellular matrix component before fragmentation measured using ANS.
Here, the fluorescence intensity of the extracellular matrix component before fragmentation measured using ANS is a fluorescence intensity measured in the same manner except that the extracellular matrix component before fragmentation (an extracellular matrix component which is used as a raw material of the fragmented extracellular matrix) is used instead of the fragmented extracellular matrix component.

[Production method of cell structure]

A production method of a cell structure according to the present embodiment includes a step of mixing cells with the fragmented extracellular matrix component obtained according to the present embodiment (mixing step); and a step of incubating the mixture obtained in the mixing step (incubation step).
In the present specification, the "cell structure" means a cell aggregate (bulky cell population) in which cells are three-dimensionally arranged through extracellular matrix components and which is artificially produced through cell culture. The shape of the cell structure is not particularly limited, and examples thereof include a sheet shape, a spherical shape, an approximately spherical shape, an ellipsoidal shape, an approximately ellipsoidal shape, a hemispherical shape, an approximately hemispherical shape, a semicircular shape, an approximately semicircular shape, a rectangular parallelepiped shape, and an approximately rectangular parallelepiped shape. The cell structure may be aggregated in a mass shape in a state where it is adhered to a support, or may be aggregated in a mass shape in a state where it is not adhered to a support.

(Mixing step)

The mixing step is a step of mixing cells with the fragmented extracellular matrix component obtained through the production method of a fragmented extracellular matrix according to the present embodiment.
Cells are not particularly limited, but cells may be derived from mammalian animals such as humans, monkeys, dogs, cats, rabbits, pigs, cattle, mice, and rats, for example. The origin of cells is also not particularly limited, but cells may be somatic cells derived from the bones, the muscles, the internal organs, the nerves, the brain, the bones, the skin, the blood, or the like, or may be reproductive cells. Furthermore, the cells may be stem cells or may be cultured cells such as primary culture cells, subcultured cells, and cell line cells.
If the fragmented extracellular matrix components are dispersed in an aqueous medium, these can be made likely to come into contact with cells in the aqueous medium to promote the formation of the cell structure.
In the mixing step, cells are mixed with and brought into contact with the fragmented extracellular matrix component in an aqueous medium. Examples of mixing steps include: a method for mixing an aqueous medium containing a fragmented extracellular matrix component with an aqueous medium containing first cells; a method for mixing an aqueous medium containing a fragmented extracellular matrix component with an aqueous medium containing cells; a method for adding cells to an aqueous medium containing a fragmented extracellular matrix component for mixing; a method for adding an aqueous medium containing a fragmented extracellular matrix component to a culture liquid containing cells for mixing; and a method for adding each of a fragmented extracellular matrix component and cells to a previously prepared aqueous medium for mixing, but the present invention is not limited thereto.
The concentration of the fragmented extracellular matrix component in the mixing step can be appropriately determined depending on the shape and thickness of a target cell structure, the size of an incubator, or the like. For example, the concentration of the fragmented extracellular matrix component in the mixing step may be 0.1 to 90 mass% or may be 1 to 30 mass%.
The amount of fragmented extracellular matrix component per $1.0 \times 10^6$ cells in the mixing step may be, for example, 0.1 to 100 mg, 0.5 to 50 mg, 0.8 to 25 mg, 1.0 to 10 mg, 1.0 to 5.0 mg, 1.0 to 2.0 mg, or 1.0 to 1.8 mg, may be greater than or equal to 0.7 mg, greater than or equal to 1.1 mg, greater than or equal to 1.2 mg, greater than or equal to 1.3 mg, or greater than or equal to 1.4 mg, or may be less than or equal to 7.0 mg, less than or equal to 3.0 mg, less than or equal to 2.3 mg, less than or equal to 1.8 mg, less than or equal to 1.7 mg, less than or equal to 1.6 mg, or less than or equal to 1.5 mg.

In the mixing step, the mass ratio of fragmented extracellular matrix components to cells (fragmented extracellular matrix components/cells) is preferably 1/1 to 1,000/1, more preferably 9/1 to 900/1, and still more preferably 10/1 to 500/1.

(Incubation step)

The incubation step is a step of incubating the mixture obtained in the mixing step. The incubation step can also be considered as a step of culturing cells in the presence of fragmented extracellular matrix components.

The method for culturing cells in the presence of fragmented extracellular matrix components is not particularly limited, and a suitable culture method can be performed depending on the types of cells to be cultured. For example, the culture temperature may be 20°C to 40°C or may be 30°C to 37°C. The pH of a medium may be 6 to 8 or may be 7.2 to 7.4. The culture time may be 1 day to 2 weeks or 1 week to 2 weeks.

The incubator (support) used in the incubation step is not particularly limited, and may be, for example, a well insert, a low adhesion plate, or a plate having a bottom surface shape such as a U-shape or a V-shape. Cells may be cultured while being adhered to a support, cells may be cultured without being adhered to a support, or may be cultured while being separated from a support in the middle of culture. In a case where cells are cultured without being adhered to a support or in a case where cells are cultured while being separated from a support in the middle of culture, a plate having a bottom surface shape such as a U-shape or a V-shape that prevents cells from adhering to a support or a low adhesion plate is preferably used.

A medium used in the incubation step is not particularly limited, and a suitable medium can be selected depending on the types of cells to be cultured. Examples of media include Eagle's MEM medium, DMEM, Modified Eagle medium (MEM), Minimum Essential medium, RPMI, and GlutaMax medium. The medium may be a medium to which serum is added, or may be a serum-free medium. The medium may be a mixed medium obtained by mixing two kinds of media with each other.

The cell density in a medium in the incubation step can be appropriately determined, for example, depending on the shape and thickness of a target cell structure, and the size of an incubator. For example, the cell density in a medium in the incubation step may be 1 to $10^8$ cells/mL, or may be $10^3$ to $10^7$ cells/mL. In addition, the cell density in a medium in the incubation step may be the same as that in an aqueous medium in the mixing step.

**Examples**

Hereinafter, the present invention will be described in more detail based on examples. However, the present invention is not limited to the following examples.

<Test Example 1>

[Production Example 1: Production of fragmented collagen]

10× PBS (manufactured by Nacalai Tesque, Inc.) was mixed with a 0.05N NaOH aqueous solution in equal amounts to prepare a neutralization solution. On ice, 4.25 mL of the neutralization solution was added at once to 17 mL of a collagen solution (porcine skin-derived collagen I solution, manufactured Nippi Inc., PSC-1-200-100), and the mixture was quickly pipetted with a pipette to neutralize the collagen solution. The pH of the collagen solution after neutralization was 7. The neutralized collagen solution was incubated at 37°C for 2 hours to form a gel. The obtained gel was frozen with liquid nitrogen, and then a solvent was removed through freeze-drying to obtain dry collagen solids (masses containing a collagen). All of the obtained dry solids were transferred to a 15 mL centrifuge tube, 10 mL of absolute ethanol was added thereto, and the mixture was dispersed and then homogenized at 30,000 rpm for 6 minutes using a homogenizer (manufactured by AS ONE Corporation, VH-10) to fragment (defibrate) the collagen. Subsequently, the fragmented collagen was precipitated through centrifugation (10,000 rpm, 3 minutes), and the supernatant ethanol (liquid component) was removed. The precipitated fragmented collagen was dried at room temperature for 3 days or dried under reduced pressure using a vacuum pump for 2 hours to further remove the ethanol (liquid component) and obtain a fragmented collagen (defibrated collagen).

[Comparative Production Example 1: Production of fragmented collagen through conventional method]

17 mL of a collagen solution (porcine skin-derived collagen I solution, manufactured by Nippi Inc., PSC-1-200-100) was freeze-dried to obtain a dried product of a collagen. 10× PBS was added to 50 mg of the obtained dried product, and the mixture was dispersed and then homogenized at 30,000 rpm for 6 minutes using a homogenizer (manufactured by AS ONE Corporation, VH-10) to fragment (defibrate) the collagen. Subsequently, the fragmented collagen was precipitated through centrifugation (10,000 rpm, 3 minutes), and the supernatant (liquid component) was removed. The precipitated fragmented collagen was freeze-dried to obtain a fragmented collagen (defibrated collagen).

[Evaluation of redispersibility]

The fragmented collagen (dried product) obtained in Production Example 1 and the fragmented collagen (dried product) obtained in Comparative Production Example 1 were evaluated for redispersibility. First, 5 mL of 1× PBS (manufactured by Nacalai Tesque, Inc., 1424924) (37°C) was added to each of the dried products, and each mixture was re-dispersed in a solution through pipetting while breaking up masses. Subsequently, after removing PBS through centrifugation (10,000 rpm, 3 minutes), each fragmented collagen was re-suspended in 5 mL of ultrapure water.

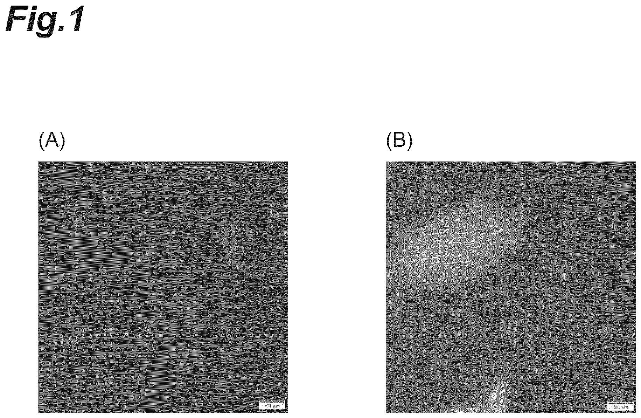

FIG. 1 illustrates optical microscope photographs of fragmented collagens re-suspended in ultrapure water. FIG. 1(A) is a fragmented collagen obtained in Production Example 1. FIG. 1(B) is a fragmented collagen obtained in Comparative Production Example 1. The fragmented collagen (Production Example 1) obtained through the production method according to the present invention could be re-dispersed through pipetting (refer to FIG. 1(A)). On the other hand, it was confirmed that the fragmented collagen (Comparative Production Example 1) obtained through the conventional production method could not be re-dispersed through pipetting and aggregates of collagen fibers remained (refer to FIG. 1(B)).

[Evaluation of three-dimensional structure of fragmented collagen]

A CD spectrum of the fragmented collagen (dried product) obtained in Production Example 1 was measured to evaluate a three-dimensional structure.
First, a part of the dried product was scraped off with a cutter, and 50 mM acetic acid was added thereto so that the concentration of the dried product was 1 mg/mL to perform dissolution overnight at 4°C. The obtained solution was diluted 200 times with 50 mM acetic acid to obtain a solution with a concentration of the dried product of 50 μg/mL. Subsequently, the obtained solution was transferred to a quartz cuvette (with an optical path length of 1 mm), and a CD spectrum (wavelength range: 200 to 250 nm) was measured using a circular dichroism spectrometer (manufactured by JASCO Corporation, J-725).

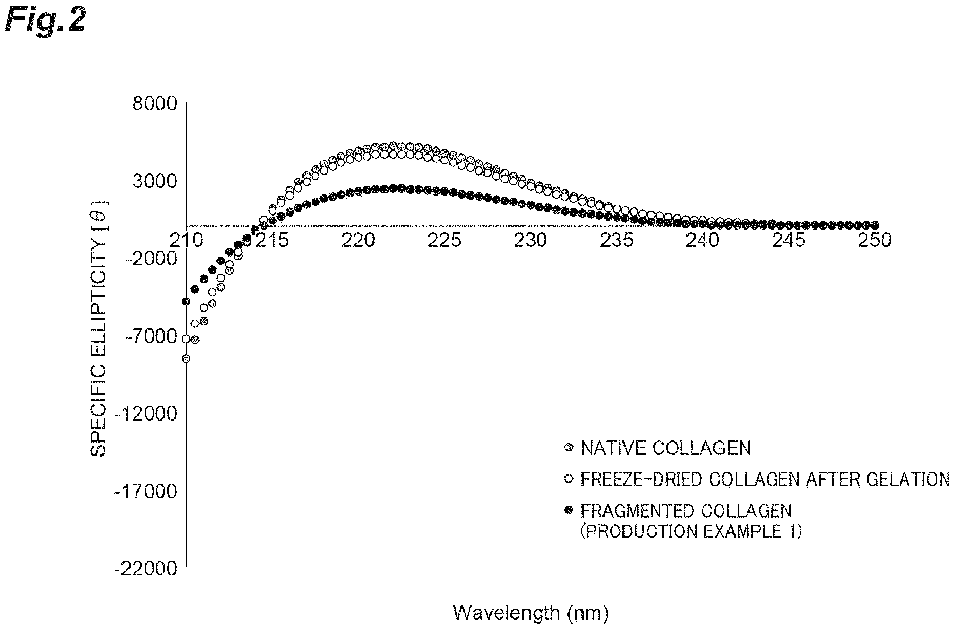

FIG. 2 is a CD spectrum of the fragmented collagen obtained in Production Example 1. FIG. 2 also concurrently shows a CD spectrum of a collagen itself (Native collagen) and a CD spectrum of a collagen freeze-dried after gelation (a freeze-dried collagen after gelation) in Production Example 1. In the CD spectra, a peak derived from a triple helix structure specific to a collagen is observed in a 220 to 225 nm region. As shown in FIG. 2, although the fragmented collagen obtained in Production Example 1 had a lower peak than the native collagen which is a standard product and the freeze-dried collagen after gelation, the peak thereof observed was derived from the triple helix structure specific to a collagen. That is, it became clear that the fragmented collagen obtained in Production Example 1 retained the triple helix structure specific to a collagen and was not denatured.

[Structural evaluation of fragmented collagen]

The structures of the fragmented collagen (dried product) obtained in Production Example 1 and the fragmented collagen (dried product) obtained in Comparative Production Example 1 were evaluated through hematoxylin-eosin (HE) staining. The HE staining was carried out on slice specimens produced from the dried products according to a usual method.

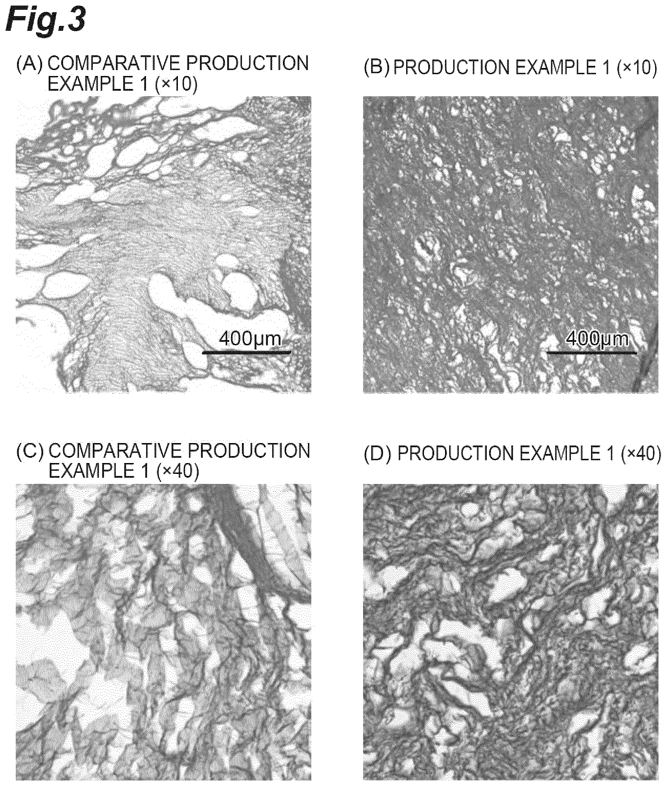

FIG. 3 illustrates photographs of hematoxylin-eosin (HE)-stained fragmented collagens. FIGS. 3(A) and 3(C) are photographs of the fragmented collagen obtained in Comparative Production Example 1 respectively imaged at 10× and 40× magnification. FIGS. 3(B) and 3(D) are photographs of the fragmented collagen obtained in Production Example 1 respectively imaged at 10× and 40× magnification. The fragmented collagen obtained in Comparative Production Example 1 forms a ribbon-like network structure (FIGS. 3(A) and 3(C)), whereas the fragmented collagen obtained in Production Example 1 had a structure in which voids between fibers were small and thin filamentous fibers were twisted together (FIGS. 3(B) and 3(D)).
The area proportion (%) of a region where extracellular matrix components were present with respect to the total area of each observation image was calculated from FIG. 3(A) (a photograph obtained by imaging the slice specimen of Comparative Production Example 1 at 10× magnification; the area of the observation image being 1820 μm (long side) × 1365 μm (short side)) and FIG. 3(B) (a photograph obtained by imaging the slice specimen of Production Example 1 at 10× magnification; the area of the observation image being 1820 μm (long side) × 1365 μm (short side)), and the results were 39.0% ± 0.5% in FIG. 3(A) (Comparative Production Example 1) and 72.3% ± 3.5% in FIG. 3(B) (Production Example 1).

Subsequently, the structures of the fragmented collagen (dried product) obtained in Production Example 1 and the fragmented collagen (dried product) obtained in Comparative Production Example 1 were further evaluated through imaging and observing with a scanning electron microscope (SEM). First, a part of each dried product was thinly scraped off with a scalpel and then attached to an electron microscope state using carbon tape. Subsequently, an osmium coater (manufactured by Shinkuu Device Co., Ltd., HPC-1SW) was used to coat the sample with osmium oxide through plasma irradiation in a vacuum for 30 seconds. The plasma irradiation was repeated three times. A scanning electron microscope (manufactured by JEOL Ltd., JSM-6701R) was used to image the obtained sample at 5.0 V and 10 μA.

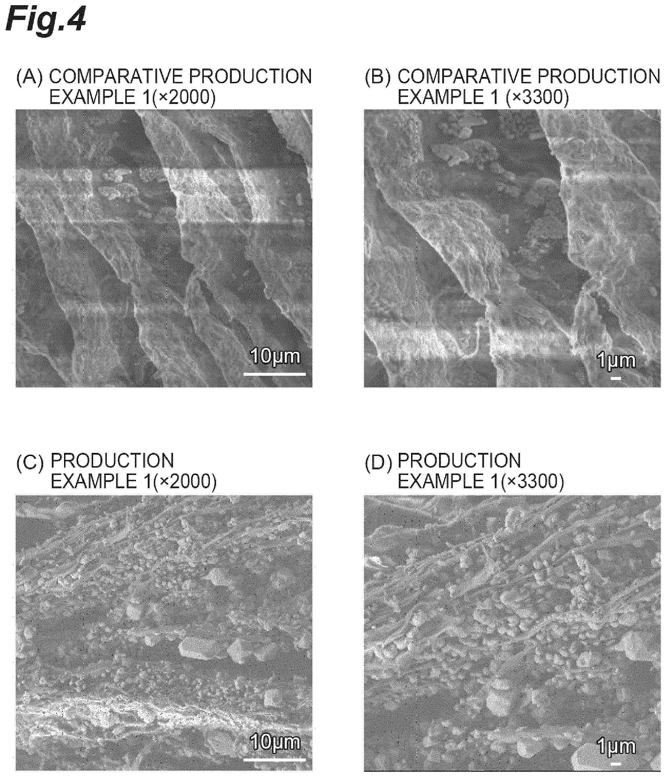

FIG. 4 illustrates photographs of fragmented collagens imaged with a scanning electron microscope. FIGS. 4(A) and 4(B) are photographs of the fragmented collagen obtained in Comparative Production Example 1 respectively imaged at 2000× and 3300× magnification. FIGS. 4(C) and 4(D) are photographs of the fragmented collagen obtained in Production Example 1 respectively imaged at 2000× and 3300× magnification. Similarly to the HE staining, a structure in which collagen fibers were stacked in a strip shape was observed in the fragmented collagen obtained in Comparative Production Example 1 (FIGS. 4(A) and 4(B)). On the other hand, it was confirmed that the fragmented collagen obtained in Production Example 1 had thin fibers overlapping each other. The average fiber diameters calculated from the obtained images were 10.6 μm for the fragmented collagen obtained in Comparative Production Example 1 and 0.6 μm for the fragmented collagen obtained in Production Example 1.

[Production Example 2: Production and evaluation of fragmented collagen]

A fragmented collagen was produced in the same procedure as in Production Example 1 except that the anhydrous ethanol as a solution for a fragmenting collagen was replaced with a 20% v/v ethanol aqueous solution (ethanol:water=20:80) (Production Example 2-1), a 50% v/v ethanol aqueous solution (ethanol:water=50:50) (Production Example 2-2), and a 70% v/v ethanol aqueous solution (ethanol:water=70:30) (Production Example 2-3).

Each yield of the fragmented collagens obtained in Production Example 1, Production Examples 2-1 to 2-3, and Comparative Production Example 1 is summarized in Table 1. The yield referred to herein is a proportion (%) of the weight of each obtained fragmented collagen (dried product) with respect to the weight of a collagen used for fragmentation.

[Table 1]

|  | Comparative Production Example 1 | Production Example 2-1 | Production Example 2-2 | Production Example 2-3 | Production Example 1 |
|---|---|---|---|---|---|
| Ethanol concentration in solution (% v/v) | 0 | 20 | 50 | 70 | 100 |
| Yield (%) | 5 | 66 | 10 | 68 | 44 |

Production Examples 2-1 to 2-3 and Production Example 1 in which collagens were fragmented in the solutions containing a polar organic solvent (ethanol) had improved yields compared to Comparative Production Example 1 in which only an aqueous solvent was used. It is inferred that the low yield in Production Example 2-2 (using 50% v/v ethanol) is due to the salt concentration resulting from salts produced through neutralization overlapping the concentration range in which a collagen is likely to be dissolved.

As a result of evaluating redispersibility in the same procedure as described in Production Example 1, all of the fragmented collagens obtained in Production Examples 2-1 to 2-3 could be re-dispersed through pipetting. FIG. 5 illustrates optical microscope photographs of fragmented collagens re-suspended in ultrapure water. FIG. 5(A) is a fragmented collagen obtained in Production Example 1. FIG. 5(B) is a fragmented collagen obtained in Production Example 2-3. FIG. 5(C) is a fragmented collagen obtained in Production Example 2-1. Although not shown in the drawings, the fragmented collagen obtained in Production Example 2-2 had similar results.

In addition, three-dimensional structures of the fragmented collagens were evaluated through in same procedure as described in Production Example 1. In the CD spectra of the fragmented collagens obtained in Production Examples 2-1 to 2-3, peaks derived from the triple helix structures specific to a collagen were all observed in a 220 to 225 nm region. That is, it became clear that the fragmented collagens obtained in Production Examples 2-1 to 2-3 retained the triple helix structures specific to a collagen and were not denatured. As an example, FIG. 6 shows the CD spectrum of the fragmented collagen obtained in Production Example 2-3. FIG. 6 also concurrently shows a CD spectrum of gelatin (without a triple helix structure) and a CD spectrum of a collagen itself (Native collagen). Although not shown in the drawing, the fragmented collagens obtained in Production Examples 2-1 and 2-2 had similar results.

[Production Example 3: Production and evaluation of fragmented collagen]

10× PBS (manufactured by Nacalai Tesque, Inc.) was mixed with a 0.05N NaOH aqueous solution in equal amounts to prepare a neutralization solution. On ice, 4.25 mL of the neutralization solution was added at once to 17 mL of a collagen solution (porcine skin-derived collagen I solution, manufactured Nippi Inc., PSC-1-200-100), and the mixture was quickly pipetted with a pipette to neutralize the collagen solution. The pH of the collagen solution after neutralization was 7. The neutralized collagen solution was incubated at 37°C for 2 hours to form a gel. The obtained gel was frozen with liquid nitrogen, and then a solvent was removed through freeze-drying to obtain dry collagen solids (masses containing a collagen). All of the obtained dry solids were transferred to a 15 mL centrifuge tube, 15 mL of acetonitrile (Production Example 3-1), acetone (Production Example 3-2), or diethyl ether (Production Example 3-3) were added thereto, and the mixture was dispersed and then homogenized at 30,000 rpm for 6 minutes using a homogenizer (manufactured by AS ONE Corporation, VH-10) to fragment (defibrate) the collagen. Subsequently, the fragmented collagen was precipitated through centrifugation (10,000 rpm, 3 minutes), and the supernatant (liquid component) was removed. The precipitated fragmented collagen was air-dried at room temperature to obtain fragmented collagens (defibrated collagens) of Production Examples 3-1 to 3-3.

The redispersibility of each of the fragmented collagens (dried products) obtained in Production Examples 3-1 to 3-3 was evaluated. 5 mL of ultrapure water was added to each of the dried products, and each mixture was re-dispersed in a solution through pipetting while breaking up masses. Subsequently, after removing the ultrapure water through centrifugation (10,000 rpm, 3 minutes), each fragmented collagen was re-suspended in 5 mL of ultrapure water. The fragmented collagens obtained in Production Examples 3-1 to 3-3 could all be re-dispersed through pipetting. FIG. 7 illustrates optical microscope photographs of fragmented collagens re-suspended in ultrapure water. FIG. 7(A) is a fragmented collagen obtained in Production Example 3-1. FIG. 7(B) is a fragmented collagen obtained in Production Example 3-2. FIG. 7(C) is a fragmented collagen obtained in Production Example 3-3.

[Reference Example 1: Observation of collagen after neutralization and after gelation]

10× PBS (manufactured by Nacalai Tesque, Inc.) was mixed with a 0.05N NaOH aqueous solution in equal amounts to prepare a neutralization solution. On ice, 4.25 mL of the neutralization solution was added at once to 17 mL of a collagen solution (porcine skin-derived collagen I solution, manufactured Nippi Inc., PSC-1-200-100), and the mixture was quickly pipetted with a pipette to neutralize the collagen solution. The pH of the collagen solution after neutralization was 7. This was freeze-dried to obtain a collagen after neutralization. In addition, the neutralized collagen solution was incubated at 37°C for 2 hours to form a gel, and this gel was freeze-dried to obtain a collagen after neutralization and gelation.

Structures of the collagen after neutralization and the collagen after neutralization and gelation were evaluated through imaging and observing with a scanning electron microscope (SEM). The imaging procedure is the same as that in Production Example 1.

FIG. 8 illustrates photographs of collagens imaged with a scanning electron microscope after neutralization and after neutralization and gelation. FIGS. 8(A) and 8(C) are photographs of a collagen respectively imaged at 500× and 2000× magnification after neutralization and gelation. FIGS. 8(B) and 8(D) are photographs of a collagen respectively imaged at 500× and 2000× magnification after neutralization. No significant difference in the structure of the collagen after neutralization and the collagen after neutralization and gelation could be confirmed. It is thought that the effect of the present invention of better redispersibility is exhibited due to a change in the structure of collagen molecules through neutralization of the collagen solution.

[Reference Example 2: Examination of pH range for gelation]

10× PBS (manufactured by Nacalai Tesque, Inc.) was mixed with a 0.05N NaOH aqueous solution in equal amounts to prepare a neutralization solution. On ice, 0.7 mL, 1.25 mL, and 1.7 mL of the neutralization solution were each added at once to 500 μL of each collagen solution dissolved in 5 mM acetic acid and neutralized through quick pipetting with a pipette. The pH of each collagen solution after neutralization was 6, 7, and 8. As a result of incubating the collagen solutions after neutralization at 37°C for 30 minutes, it was confirmed that all collagen solutions were gelled. FIG. 9 is a photograph of gelled collagen solutions.

[Production Example 4: Production of cell structure]

Using the fragmented collagens obtained in Production Example 1 and Comparative Production Example 1, cell structures were produced.

5 mL of 1× PBS (manufactured by Nacalai Tesque, Inc., 1424924) (37°C) was added to the entire amount of the fragmented collagen (dried product) obtained in Production Example 1, and the mixture was re-dispersed in a solution through pipetting while breaking up masses. Subsequently, after removing the PBS through centrifugation (10,000 rpm, 3 minutes), the fragmented collagen was suspended in DMEM medium (manufactured by Nacalai Tesque, Inc., 845816) containing 5 mL of 10% serum (manufactured by Invitrogen, 10270-106). 1 mL of the suspension was transferred to a 1.5 mL microtube. $2.0 \times 10^6$ normal human dermal fibroblasts (NHDF) (manufactured by Lonza KK., CC-2509) and $1.0 \times 10^6$ human umbilical vein endothelial cells (HUVEC) (manufactured by Lonza KK., C2517A) peeled off with 0.025% trypsin (0.01% EDTA) (manufactured by FUJIFILM Wako Pure Chemical Corporation, 207-19183) were dispensed into the microtube, and the mixture was centrifuged at 10,000 rpm for 1 minute. A prescribed amount thereof was dispensed into a 24-well insert (manufactured by Corning Inc., #3470), and the insert was centrifuged at 1,100 × g for 15 minutes. Subsequently, the insert was transferred to a 6-well culture plate, and culture was performed for 7 days in a medium containing a 1:1 mixture of EGM (registered trademark)-2MV BulletKit (registered trademark) (manufactured by Lonza KK., CC-3202) and 10% serum-containing DMEM medium. After culture, the resultant was fixed in a 4% paraformaldehyde/phosphate buffer overnight at room temperature and embedded in paraffin, and then thin slice specimens were subjected to HE staining and CD31 staining. A cell structure for the fragmented collagen (dried product) obtained in Comparative Production Example 1 was produced in the same procedure as described above and subjected to HE staining.

FIG. 10 illustrates photographs of cell structures stained with hematoxylin and eosin (HE) (FIGS. 10(A) and 10(C)) and immunostained with CD31 (FIG. 10(B)). FIGS. 10(A) and 10(B) are cell structures produced using the fragmented collagen obtained in Production Example 1. FIG. 10(C) is a cell structure produced using the fragmented collagen obtained in Comparative Production Example 1. As shown in FIG. 10, although the cell structure produced using the fragmented collagen obtained in Production Example 1 showed some collagen aggregation, tissue thereof constructed was almost equivalent to that of the cell structure (conventional method) produced using the fragmented collagen obtained in Comparative Production Example 1 (FIGS. 10(A) and 10(C)). In addition, in the cell structure produced using the fragmented collagen obtained in Production Example 1, no noticeable cell death was observed, and CD31 staining revealed that blood vessels with a luminal structure were constructed (FIG. 10(B)).

<Test Example 2>

[Material] A collagen I solution made from porcine skin (NIPPI/PSC-1-200-100) was used as a collagen.

Cells listed in Table 2 were used as cells.

[Table 2]

| Cell type | Manufacturer | Product number |
|---|---|---|
| Normal human dermal fibroblasts | Lonza KK. | CC-2509 |
| Human umbilical vein endothelial cells | Lonza KK. | C2517A |

Reagents listed in Table 3 were used as reagents.

[Table 3]

| Reagent name | Manufacturer | Product number |
|---|---|---|
| DMEM Medium | Nacalai Tesque, Inc. | 845816 |
| EGM™-2MV BulletKit™ | Lonza KK. | CC-3202 |
| Fetal Bovine Serum | Invitrogen | 10270-106 |
| Antibiotic-antifungal mixed solution | Nacalai Tesque, Inc. | 289254 |
| 1× PBS | Nacalai Tesque, Inc. | 1424924 |
| Biochemical trypsin | FUJIFILM Wako Pure Chemical Corporation | 207-19183 |
| Carrazzi's hematoxylin | Muto Pure Chemicals Co., Ltd. | 30022 |
| Monoclonal mouse anti-human CD31 | Dako | MO82301-2 |

[Experimental procedure: Preparation of fragmented collagen] Production Example 5-1

10× PBS was mixed with 0.05N NaOH in equal amounts to prepare a neutralization solution. On ice, 4.25 mL of the neutralization solution was added at once to 17 mL of a collagen solution and neutralized through quick pipetting with a pipette. The neutralized collagen solution was incubated at 37°C for 2 hours to form a gel. The obtained gel was frozen with liquid nitrogen, and then freeze-dried for at least 48 hours to obtain dry collagen solids (masses containing a collagen) which was freeze-dried after gelation.

All of the obtained dry solids were transferred to a 15 mL centrifuge tube, 15 mL of absolute ethanol (ethanol:water=100:0) was added thereto, and the collagen was dispersed and then homogenized for 6 minutes using a homogenizer with a power of 6 to fragment (defibrate) the collagen. Subsequently, the fragmented collagen was precipitated through centrifugation at 10,000 rpm, for 3 minutes, and the supernatant liquid component was removed. 15 mL of ultrapure water was added to the precipitated fragmented collagen to obtain a liquid containing the fragmented collagen and the ultrapure water. The liquid was frozen using liquid nitrogen in a state where the fragmented collagen was dispersed through pipetting, and then freeze-dried for at least 48 hours. As a result, a fragmented collagen of Production Example 5-1 was obtained as a dry solid.

Production Examples 5-2, 5-3, and 5-4

Fragmented collagens of Production Examples 5-2, 5-3, and 5-4 were produced in the same procedure as in Production Example 5-1 except that a 20% v/v ethanol aqueous solution (ethanol:water=20:80) (Production Example 5-2), a 50% v/v ethanol aqueous solution (ethanol:water=50:50) (Production Example 5-3), and a 70% v/v ethanol aqueous solution (ethanol:water=70:30) (Production Example 5-4) were used as solutions for fragmenting a collagen.

Comparative Production Example 5-1

A fragmented collagen of Comparative Production Example 5-1 was obtained in the same manner as in Production Example 5-1 except that water (ethanol:water=0:100) was used as a solution for fragmenting a collagen.

Reference Example 3

10× PBS (manufactured by Nacalai Tesque, Inc.) was mixed with a 0.05N NaOH aqueous solution in equal amounts to prepare a neutralization solution. On ice, 4.25 mL of the neutralization solution was added at once to 17 mL of a collagen solution (porcine skin-derived collagen I solution, manufactured Nippi Inc., PSC-1-200-100), and the mixture was quickly pipetted with a pipette to neutralize the collagen solution. The pH of the collagen solution after neutralization was 7. The neutralized collagen solution was incubated at 37°C for 2 hours to form a gel. The obtained gel was frozen with liquid nitrogen, and then a solvent was removed through freeze-drying to obtain dry collagen solids (masses containing a collagen). All of the obtained dry solids were transferred to a 15 mL centrifuge tube, 10 mL of a 70% v/v ethanol aqueous solution (ethanol:water=70:30) was added thereto, and the mixture was dispersed and then homogenized at 30,000 rpm for 6 minutes using a homogenizer (manufactured by AS ONE Corporation, VH-10) to fragment (defibrate) the collagen. Subsequently, the fragmented collagen was precipitated through centrifugation (10,000 rpm, 3 minutes), and the supernatant ethanol (liquid component) was removed. The precipitated fragmented collagen was dried at room temperature for 3 days or dried under reduced pressure using a vacuum pump for 2 hours to further remove the ethanol (liquid component) and obtain a fragmented collagen (defibrated collagen) of Reference Example 3.

[Production Example 5-5: Production of finer fragmented collagen through ultrasonic crushing]

A liquid containing a fragmented collagen and ultrapure water was obtained in the same manner as in Production Example 5-1 except that a 70% v/v ethanol aqueous solution (ethanol:water=70:30) was used as a solution for fragmenting a collagen. Ultrasonic irradiation for 20 seconds and cooling for 30 seconds were repeated 99 times using a fully automatic ultrasonic crusher (BIORUPTER II, BM Equipment Co., Ltd.) in a state where the fragmented collagen was suspended in the ultrapure water, and the fragmented collagen was subjected to ultrasonic crushing treatment. The fragmented collagen after ultrasonic crushing treatment was freeze-dried while being dispersed in pure water to obtain a fragmented collagen of Production Example 5-5 as a dry solid.

[Production of Cell structure (three-dimensional cell structure)]

The fragmented collagen of Production Example 5-4, the fragmented collagen of Reference Example 3, and the fragmented collagen of Production Example 5-5 were respectively used for constructing cell structures.

8 mg of dry solids of each fragmented collagen was weighed into a microtube and pipetted with 500 μL of ultrapure water to disperse the fragmented collagen, thereby obtaining a fragmented collagen dispersion liquid. The fragmented collagen dispersion liquid was centrifuged at 10,000 rpm for 1 minute to precipitate the fragmented collagen, a liquid component of a supernatant was removed, $2.0 \times 10^6$ NHDFs and $1.0 \times 10^6$ HUVECs which were peeled off with 0.025% trypsin and 0.01% EDTA and suspended in a complete medium were dispensed into the microtube, and the mixture was centrifuged at 10,000 rpm for 1 minute to obtain a cell-containing liquid. A prescribed amount of the cell-containing liquid was dispensed into a 24-well insert (Corning Inc., #3470), and the insert was centrifuged at 1,100 × g for 15 minutes. The insert was transferred to a 6-well culture plate, and the cells were cultured for 7 days in a medium containing a 1:1 mixture of EGM™-2MV BulletKit™ and 10% serum-containing DMEM. The tissue construct obtained through culture was fixed in a 4% paraformaldehyde/phosphate buffer overnight at room temperature and embedded in paraffin, and then thin slice specimens were subjected to hematoxylin-eosin staining (HE staining).

[Measurement of CD spectrum]

Some of dry solids of each fragmented collagen was scraped off, 50 mM acetic acid was added thereto so that the concentration of the dry solids was 1 mg/mL to perform dissolution overnight at 4°C and prepare a fragmented collagen solution. The fragmented collagen solution was diluted 200 times with 50 mM acetic acid so that the concentration of the collagen was 50 μg/mL to obtain a solution for measurement. The solution for measurement was transferred to a quartz cuvette with an optical path length of 1 mm, and a CD spectrum was measured at 200 nm to 250 nm using a circular dichroism spectrometer (JASCO Corporation, J-725).

[Evaluation of fragmented collagen]

FIG. 11 is a photograph showing an evaluation result of redispersibility of the fragmented collagen of Production Example 5-4 produced using a 70% v/v ethanol aqueous solution (ethanol:water=70:30) as a solution for fragmenting a collagen. It was confirmed that the fragmented collagen of Production Example 5-4 was almost completely dispersed through pipetting with ultrapure water at 37°C for about 1 minute (FIG. 11). FIG. 12 is a photograph of the fragmented collagen of Production Example 5-4 imaged with a phase-contrast microscope. It was also confirmed from the phase-contrast micrograph that the collagen was fragmented (microfibered) (FIG. 12).

The method of obtaining a fragmented collagen through performing freeze-drying treatment without replacing a liquid component with ultrapure water after a collagen was fragmented in a solution containing ethanol when producing a fragmented collagen (Reference Example 3) had disadvantages that the dried product of the fragmented collagen will become hard and require about 5 minutes to be re-dispersed and that the time required for drying to obtain a dried product will be significantly long (about 2 weeks) if ethanol concentration in the solution for fragmenting a collagen is high. On the other hand, the methods for fragmenting a collagen in a solution containing ethanol, replacing a liquid component with ultrapure water, and then performing freeze-drying treatment to obtain a fragmented collagen did not have such disadvantages.

[Evaluation of modification of fragmented collagen]

FIG. 13 shows measurement results of CD spectra of gelatin, a raw material collagen (non-fragmented collagen, Native Collagen), and the fragmented collagen of Production Example 5-4.

As shown in FIG. 13, although a slight decrease in the peak value from the raw material collagen was observed, a triple helix-specific peak around 220 nm was confirmed. Therefore, it became clear that the fragmented collagen was not denatured.

FIG. 14 shows measurement results of CD spectra of gelatin, a raw material collagen (Native Collagen), and fragmented collagens of production examples and a comparative production example.

As shown in FIG. 14, no denaturation was observed under any of the conditions. As shown in FIG. 14, the peaks were reduced when the concentration of ethanol in an ethanol aqueous solution was 20% v/v and 50% v/v compared to when the concentration of ethanol in an ethanol aqueous solution was 0% v/v, 70% v/v, and 100% v/v. In the measurement of the CD spectra, a collagen in a solution state was used as the native collagen, and ones obtained by dissolving dried products were used as the fragmented collagens. A collagen in a dry state is likely to absorb moisture, and its weight changes even during measurement. Therefore, the concentration of a solution used for measurement may be somewhat lower than that of a collagen in a solution from the beginning. However, since there is no difference in the measurement conditions between the production examples and the comparative production examples, the difference in peak amount between the production examples and the comparative production examples actually indicates a difference in the amount of the triple helix structure in the samples. Ethanol itself has an effect of denaturing and aggregating proteins, but this is because ethanol changes three-dimensional structures of the proteins which is composed of hydrogen bonds. Therefore, the specific amount of hydrogen bonds that ethanol changes depends on, for example, the types of proteins and the number of hydrogen bonds between or within molecules. The conditions with lower peaks (ethanol concentrations of 20% v/v and 50% v/v) are conditions with more moisture during defibration than the other conditions. Accordingly, it is thought that more hydrogen bonds were formed in the collagens, and the degree of the structures changed by the ethanol was considered to be greater than that under a condition with a high ethanol concentration or a condition with no ethanol at all.

It was evaluated whether the yield of an obtained fragmented collagen changes when a solution for fragmenting a collagen does not contain ethanol, when the concentration of ethanol in a solution for fragmenting a collagen is low (20% v/v), when the concentration of ethanol in a solution for fragmenting a collagen is high (70% v/v, 100% v/v). The results are shown in Table 4. The yield was calculated by measuring a weight $V0$ of a collagen used for fragmentation and a weight $V1$ of an obtained fragmented collagen (dried product) according to Equation (1) below.

$$Yield(\%)=V1/V0\times100 \qquad (1)$$

[Table 4]

| Ethanol concentration | 0% v/v | 20% v/v | 70% v/v | 100% v/v |
|---|---|---|---|---|
| Yield | 25.7% | 86.5% | 75.3% | 76.3% |

As shown in Table 4, the yield was improved when the concentration of ethanol in a solution for fragmenting a collagen is low (20% v/v) and when the concentration of ethanol in a solution for fragmenting a collagen is high (70% v/v, 100% v/v) compared to when a solution for fragmenting a collagen does not contain ethanol.

[Evaluation of cell structure]

FIG. 15 illustrates photographs showing cell structures stained with hematoxylin and eosin (HE) and immunostained with CD31 which are produced using the fragmented collagen (example) obtained through the method of Production Example 5-1 or the fragmented collagen (reference example) obtained through the method of Reference Example 3. The fragmented collagen of the production example was organized without being dissolved even if a medium in which cells were suspended was used for replacement.

Looking at the HE-stained images of the obtained tissues, no significant morphological difference was observed between the fragmented collagen obtained through the method of Production Example 5-1 and the fragmented collagen obtained through the method of Reference Example 3. Furthermore, the presence of a vascular network with a luminal structure was also observed by CD31 staining, indicating that there was no difference in the three-dimensional tissue image obtained even in a case where the fragmented collagen of the production example was used.

[Ultrasonic crushing of fragmented collagen]

In conventional methods that do not use organic solvents during defibration, when ultrasonic crushing (ultrasonic defibration) is performed while cooling, collagen dissolves during defibration. Therefore, to obtain a finer fragmented collagen through ultrasonic crushing, it was necessary to prevent dissolution through thermal cross-linking treatment. However, because collagens may be denatured through thermal cross-linking treatment, there was a disadvantage that tissues obtained using thermally cross-linked fragmented collagens are significantly different from tissues produced without performing thermal cross-linking treatment. Since the fragmented collagen of Production Example 5-5 defibrated using a homogenizer in ethanol has a property of not dissolving easily even if a liquid component is replaced with ultrapure water after fragmentation, it could be further defibrated by ultrasound as it is.

FIG. 16 shows micrographs of the fragmented collagen of Production Example 5-5. As shown in FIG. 16, although fibers before being ultrasonically crushed are defibrated, individual fibers themselves are entangled with each other. Fibers after being ultrasonically crushed were found to be separated and dispersed in a liquid one by one, although their length was not significantly different from the previous ones.

FIG. 17 illustrates photographs of cell structures stained with hematoxylin and eosin (HE) and immunostained with CD31 which are produced using the fragmented collagen of Production Example 5-5. Before ultrasonic crushing, solidified portions (circles) of collagens were observed and the distribution of cells was uneven. However, after ultrasonic crushing, no collagen masses were observed and the cells were evenly distributed.

[Evaluation of fragmented collagen produced using hydrophilic organic solvent other than ethanol]

Fragmented collagens of Production Example 5-6 (acetone), Production Example 5-7 (acetonitrile), and Production Example 5-8 (diethyl ether) were produced using acetone, acetonitrile, and diethyl ether instead of a 70% v/v ethanol aqueous solution. The concentration of a polar organic solvent used instead of the 70% v/v ethanol aqueous solution was 100% v/v.

FIG. 18 shows photographs of collagens defibrated using various polar organic solvents. Fiber formation was possible even in a case where polar organic solvents other than ethanol were used.

Even in Productions 5-6 to 5-8 in which polar organic solvents other than the 70% v/v ethanol aqueous solution were used, rapid redispersibility was confirmed similarly to the case where the 70% v/v ethanol aqueous solution was used.

[Hydrophobicity evaluation by ANS-8]

The fragmented collagen of Production Example 5-4, the fragmented collagen of Reference Example 3, and a freeze-dried collagen solution were each added to $1\times$ PBS to a concentration of 5 mg/mL and dissolved in a refrigerator for 4 days to prepare various collagen solutions.

ANS-8 Mg (magnesium(II) 8-anilino-1-naphthalenesulfonate) (Tokyo Chemical Industry Co., Ltd., product code: A5353) was dissolved in $1\times$ PBS to a concentration of 20 $\mu$M to prepare an ANS-Mg aqueous solution. 200 $\mu$E of the ANS-Mg aqueous solution was added to 500 $\mu$L of each collagen solution to cause a reaction under a light shielding condition at room temperature (25°C) for 1 hour. Using each of the obtained reaction solutions, spectra of the samples were measured with a spectrophotometer (Nanodrop 3300, Thermo Fischer Scientific Inc.) at a wavelength of 380 nm.

Measurement results of the spectra are shown in FIG. 19. In FIG. 19, "New Method CMF" indicates a measurement result in a case where the fragmented collagen of Production Example 5-4 was used, "Normal CNW" indicates a measurement result in a case where the fragmented collagen of Reference Example 3 was used, and "Native collagen" indicates a measurement result in a case where a freeze-dried collagen solution was used.

**Claims**

1. A production method of a fragmented extracellular matrix component, comprising:

   a step of neutralizing a solution in which an extracellular matrix component is dissolved;
   a step of obtaining a mass containing the extracellular matrix component by removing a solvent through freeze-drying treatment after the neutralization step;
   a step of obtaining a liquid containing the fragmented extracellular matrix component by dispersing the mass in a solution containing a polar organic solvent and fragmenting the extracellular matrix component in the solution; and
   a step B of removing a liquid component from the liquid containing the fragmented extracellular matrix component.

2. The production method according to claim 1, further comprising:

   a step of replacing the liquid component in the liquid obtained in the step of obtaining the liquid with water after the step of obtaining the liquid and before the step of removing the liquid component,
   wherein, in the step of removing the liquid component, the liquid component is removed from the liquid containing the fragmented extracellular matrix component through freeze-drying treatment.

3. The production method according to claim 2, further comprising:
   a step of subjecting the liquid containing the fragmented extracellular matrix component to ultrasonic crushing treatment after the step of performing replacement with the water and before the step of removing the liquid component.

4. The production method according to any one of claims 1 to 3, further comprising:
   a step of gelling the neutralized solution after the neutralization step and before the step of obtaining the mass.

5. The production method according to claim 4,
   wherein the mass is a porous body.

6. The production method according to any one of claims 1 to 5,

wherein the fragmentation comprises defibration of an extracellular matrix component.

7. The production method according to any one of claims 1 to 6,
   wherein the concentration of the polar organic solvent in the solution containing the polar organic solvent is 20% v/v to 100% v/v.

8. The production method according to any one of claims 1 to 7,
   wherein the polar organic solvent comprises ethanol.

9. The production method according to any one of claims 1 to 8, further comprising:
   a step of dispersing a solid matter obtained in the step of removing the liquid component in an aqueous medium.

10. The production method according to any one of claims 1 to 9,
    wherein the extracellular matrix component is a collagen.

11. The production method according to claim 10,
    wherein the fragmented extracellular matrix component has a triple helix structure specific to a collagen.

12. A production method of a cell structure, comprising:

    a step of mixing cells with the fragmented extracellular matrix component obtained through the production method according to any one of claims 1 to 11; and
    a step of incubating the mixture obtained in the mixing step.

13. A dried product of fragmented extracellular matrix components, comprising:
    stacked fibrous extracellular matrix components having an average diameter of 0.08 $\mu$m to 2.0 $\mu$m.

14. The dried product of fragmented extracellular matrix components according to claim 13,
    wherein, in an observation image acquired by preparing a slice specimen and observing a rectangular region of the slice specimen with a long side of 1820 $\mu$m and a short side 1365 $\mu$m using a microscope, an area of a region where the extracellular matrix components are present in the area of the observation image is 40% to 100%.

15. The dried product of fragmented extracellular matrix components according to claim 13 or 14,
    wherein the fragmented extracellular matrix components are fragmented collagens.

16. A fragmented extracellular matrix component, having a peak within a wavelength range of 400 nm to 550 nm as measured by fluorescence intensity using 1,8-anilinonaphthalene sulfonic acid (ANS).

## Fig.1

(A)

(B)

**Fig.2**

Legend:
- NATIVE COLLAGEN
- FREEZE-DRIED COLLAGEN AFTER GELATION
- FRAGMENTED COLLAGEN (PRODUCTION EXAMPLE 1)

Y-axis: SPECIFIC ELLIPTICITY [θ] (8000, 3000, -2000, -7000, -12000, -17000, -22000)

X-axis: Wavelength (nm) (210, 215, 220, 225, 230, 235, 240, 245, 250)

# Fig.3

(A) COMPARATIVE PRODUCTION
    EXAMPLE 1 (×10)

(B) PRODUCTION EXAMPLE 1 (×10)

(C) COMPARATIVE PRODUCTION
    EXAMPLE 1 (×40)

(D) PRODUCTION EXAMPLE 1 (×40)

*Fig.4*

(A) COMPARATIVE PRODUCTION
     EXAMPLE 1(×2000)

10μm

(B) COMPARATIVE PRODUCTION
     EXAMPLE 1 (×3300)

1μm

(C) PRODUCTION
     EXAMPLE 1(×2000)

10μm

(D) PRODUCTION
     EXAMPLE 1(×3300)

1μm

EP 4 328 233 A1

# Fig.5

(A) 100% ETHANOL (×10)    (B) 70% ETHANOL (×10)    (C) 20% ETHANOL (×10)

Fig.6

EP 4 328 233 A1

*Fig.7*

(A) ACETONITRILE      (B) ACETONE      (C) DIETHYL ETHER

# Fig.8

(A) AFTER NEUTRALIZATION AND
GELATION (×500)

(B) AFTER NEUTRALIZATION (×500)

(C) AFTER NEUTRALIZATION AND
GELATION (×2000)

(D) AFTER NEUTRALIZATION (×2000)

EP 4 328 233 A1

**Fig.9**

pH6

pH7

pH8

29

# Fig.10

(A)

X10  HE STAINING

400μm

(B)

X40  CD31 STAINING

100μm

(C)

X10 HE STAINING

400μm

Fig.11

*Fig.12*

## *Fig.13*

## Fig.14

# Fig.15

REFERENCE EXAMPLE          EXAMPLE

HE STAINING
x10

400μm          400μm

CD31 STAINING
x40

100μm          100μm

Fig.16

# *Fig.17*

|  | ULTRASONICALLY CRUSHED | NO ULTRASONIC CRUSHING |
|---|---|---|
| HE STAINING x10 | 400μm | 400μm |
| CD31 STAINING x40 | 100μm | 100μm |

*Fig.18*

ACETONE                    ACETONITRILE                    DIETHYL ETHER

100μm                      100μm                           100μm

Fig.19

## INTERNATIONAL SEARCH REPORT

| International application No. |
|---|
| **PCT/JP2022/026062** |

### A. CLASSIFICATION OF SUBJECT MATTER

*C07K 1/02*(2006.01)i; *C07K 1/14*(2006.01)i; *C07K 5/09*(2006.01)i; *C12N 5/071*(2010.01)i; *C12N 1/00*(2006.01)i; *C07K 14/78*(2006.01)i
FI: C07K14/78; C07K1/02; C07K5/09; C12N5/071; C12N1/00 F; C12N1/00 Z; C07K1/14

According to International Patent Classification (IPC) or to both national classification and IPC

### B. FIELDS SEARCHED

Minimum documentation searched (classification system followed by classification symbols)

C07K1/02; C07K1/14; C07K5/09; C12N5/071; C12N1/00; C07K14/78

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | WO 2012/015055 A1 (NIPPI, INC.) 02 February 2012 (2012-02-02) claims 1-9, example 1 | 13-16 |
| Y | | 1-16 |
| Y | WO 2019/208832 A1 (TOPPAN PRINTING CO., LTD.) 31 October 2019 (2019-10-31) paragraphs [0111]-[0113] | 1-16 |
| A | WO 2019/189786 A1 (TOPPAN PRINTING CO., LTD.) 03 October 2019 (2019-10-03) claims 1-12, paragraphs [0034], [0076]-[0078] | 1-16 |
| Y | WO 2019/208831 A1 (TOPPAN PRINTING CO., LTD.) 31 October 2019 (2019-10-31) claims 1-11, paragraphs [0024], [0033], [0098] | 1-16 |
| A | TOPRAGGALEH, T. R. et al. A testis-derived macroporous 3D scaffold as a platform for the generation of mouse testicular organoids. Biomaterials Science. 2019, vol. 7, pages 1422-1436, DOI: 10.1039/c8bm01001c abstract | 1-16 |

☐ Further documents are listed in the continuation of Box C.  ☑ See patent family annex.

| | | |
|---|---|---|
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **18 August 2022** | **30 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/026062**

| Patent document cited in search report | | | Publication date (day/month/year) | Patent family member(s) | | | Publication date (day/month/year) |
|---|---|---|---|---|---|---|---|
| WO | 2012/015055 | A1 | 02 February 2012 | US | 2013/0190479 | A1 | |
| | | | | claims 1-9, example 1 | | | |
| | | | | EP | 2599820 | A1 | |
| | | | | CN | 103080194 | A | |
| | | | | KR | 10-2013-0066664 | A | |
| WO | 2019/208832 | A1 | 31 October 2019 | US | 2021/0238542 | A1 | |
| | | | | paragraphs [0132]-[0134] | | | |
| | | | | EP | 3786179 | A1 | |
| | | | | CN | 111902424 | A | |
| WO | 2019/189786 | A1 | 03 October 2019 | (Family: none) | | | |
| WO | 2019/208831 | A1 | 31 October 2019 | US | 2021/0115377 | A1 | |
| | | | | claims 1-15, paragraphs [0047], [0056], [0127] | | | |
| | | | | EP | 3786175 | A1 | |
| | | | | CN | 112041332 | A | |

Form PCT/ISA/210 (patent family annex) (January 2015)

**REFERENCES CITED IN THE DESCRIPTION**

**Patent documents cited in the description**

- WO 2018143286 A